(19) Europäisches Patentamt
European Patent Office
Office européen des brevets

(11) Numéro de publication: **0 150 139**
**B1**

(12) # FASCICULE DE BREVET EUROPÉEN

(45) Date de publication du fascicule du brevet: **18.05.88**

(51) Int. Cl.[4]: **C 07 D 209/08**, C 07 D 209/34, A 61 K 31/40

(21) Numéro de dépôt: **85400025.4**

(22) Date de dépôt: **08.01.85**

(54) **Dérivés de l'indole éthénylphénol, leurs sels, procédé de préparation, application à titre de médicaments,et compositions les renfermant.**

(30) Priorité: **13.01.84 FR 8400492**

(43) Date de publication de la demande: **31.07.85 Bulletin 85/31**

(45) Mention de la délivrance du brevet: **18.05.88 Bulletin 88/20**

(84) Etats contractants désignés: **AT BE CH DE FR GB IT LI LU NL SE**

(56) Documents cité:
**EP-A-0 089 426**
**FR-A-2 528 043**
**US-A-4 333 951**

(73) Titulaire: **ROUSSEL- UCLAF, 35, boulevard des Invalides, F-75007 Paris (FR)**

(72) Inventeur: **Guillaume, Jacques, 15, av. du Belvédère (Appt. 1904), F-93310- Le- Pre- Saint-Gervais (FR)**
Inventeur: **Clemence, François, 2, rue Turgot, F-75009 Paris (FR)**
Inventeur: **Brown, Neil Leslie, 12, rue Jaucourt, F-75012 Paris (FR)**

(74) Mandataire: **Tonnellier, Marie- José, Département des Brevets ROUSSEL UCLAF B.P. no 9, F-93230 Romainville (FR)**

EP 0 150 139 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne de nouveaux dérivés de l'indol éthényl phénol ainsi que leurs sels, le procédé de préparation, l'application à titre de médicaments de ces nouveaux dérivés et les compositions les renfermant.

L'invention a pour objet de nouveaux dérivés de l'indol éthényl phénol, ainsi que leurs sels d'addition avec les acides minéraux ou organiques, caractérisés en ce qu'ils répondent à la formule générale (I):

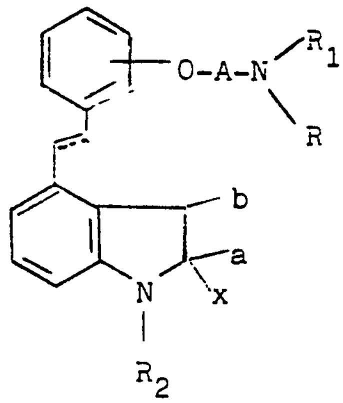

(I)

dans laquelle

R et $R_1$ représentent, indépendamment l'un de l'autre, un atome d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone éventuellement substitué par 1, 2 ou 3 radicaux choisis parmi le groupe des halogènes, méthyle, éthyle, méthoxy, éthoxy, trifluorométhyle, méthylthio, amino et nitro, ou
R et $R_1$ forment ensemble un hétérocycle saturé choisi parmi les cycles pyrrolidino, pipéridino, morpholino, pipérazinyle, méthyl pipérazinyle, éthyl pipérazinyle ou propyl pipérazinyle,
a représente ensemble avec x une fonction oxo, ou représente ensemble avec b une liaison carbone-carbone,
x représente un atome d'hydrogène, ou ensemble avec a une fonction oxo,
b représente un atome d'hydrogène, ou ensemble avec a représente une liaison carbone-carbone,
le pointillé représente la présence éventuelle d'une liaison carbone-carbone,
A représente une chaîne

$-CH_2-CH-CH_2-$
$\quad\quad\ |$
$\quad\quad OH$

ou $-(CH_2)_n$
dans laquelle n peut prendre les valeurs 2, 3, 4 ou 5 et $R_2$ représente un atome d'hydrogène, un radical alkyle linéaire renfermant de 1 à 5 atomes de carbone, ou un radical alkyle ramifié renfermant de 3 à 5 atomes de carbone, les dérivés de formule (I) étant de configuration trans, lorsque le pointillé représente une liaison carbone-carbone, étant entendu que dans le cas où A représente une chaîne

$CH_2-CH-CH_2-$,
$\quad\quad |$
$\quad\ OH$

R et $R_1$ ne peuvent pas représenter en même temps un atome d'hydrogène.

Dans la formule générale (I) et dans ce qui suit, le terme radical alkyle linéaire renfermant de 1 à 5 atomes de carbone désigne, de préférence, un radical méthyle, éthyle ou propyle; le terme radical alkyle ramifié renfermant de 3 à 5 atomes de carbone désigne, de préférence, un radical isopropyle ou tert-butyle, le terme cycloalkyle renfermant de 3 à 7 atomes de carbone désigne, de préférence, un radical cyclopentyl; le terme radical cycloalkylalkyle renfermant de 4 à 7 atomes de carbone désigne, de préférence, un radical cyclopropylméthyle; le terme radical aralkyle renfermant de 7 à 12 atomes de carbone désigne, de préférence, un radical benzyle ou phénéthyle, éventuellement substitué par 1, 2 ou 3 radicaux choisis parmi le groupe des halogènes, méthyle, éthyle, méthoxy, éthoxy, trifluorométhyle, méthylthio, amino et nitro.

Les sels d'addition avec les acides minéraux ou organiques peuvent être, par exemple, les sels formés avec les acides chlorhydrique, bromhydrique, nitrique, sulfurique, phosohorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou

paratoluène sulfoniques et arylcarboxyliques.

Parmi les produits objet de l'invention, on peut citer notamment les dérivés répondant à la formule (I) ci-dessus, ainsi que leurs sels d'addition avec les acides minéraux ou organiques, caractérisés en ce que, dans ladite formule (I), $R_2$ représente un atome d'hydrogène, A représente une chaîne

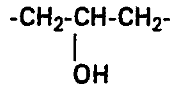

et R et $R_1$ représentent un atome d'hydrogène ou un radical alkyle, étant entendu que R et $R_1$ ne peuvent pas représenter en même temps un atome d'hydrogène.

Parmi les produits, objet de l'invention, on peut encore citer les dérivés répondant à la formule (I) ci-dessus, ainsi que leurs sels d'addition avec les acides minéraux ou organiques, caractérisés en ce que, dans ladite formule (I), $R_2$ représente un atome d'hydrogène, A représente une chaîne $-(CH_2)_n-$, n étant défini comme précédemment et R et $R_1$ représentent un atome d'hydrogène ou un radical alkyle.

Parmi les produits, objet de l'invention, on peut encore citer les dérivés répondant à la formule (I) ci-dessus, ainsi que leurs sels d'addition avec les acides minéraux ou organiques, caractérisés en ce que, dans ladite formule (I), la chaîne latérale

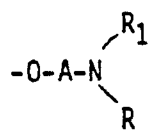

est fixée en position ortho.

Parmi les différents produits préférés ci-dessus, on peut citer plus particulièrement les dérivés caractérisés en ce que, dans ladite formule (I), R représente un atome d'hydrogène et a et b représentent ensemble une liaison carbone-carbone, et tout particulièrement,

- le 1-/(1,1-diméthyl éthyl) amino/-3-/4-(2/-(1H-indol-4-yl) éthényl/phénoxy/2-propanol,
- le 1-/(1,1-diméthyl éthyl)amino/-3-/2/2-(1H-indol-4-yl) éthyl/phénoxy/2-propanol,
- la 1,3-dihydro 4-/2-/2-/3-/(1,1-diméthyl éthyl)amino/2-hydroxypropoxy/phényl/éthényl/2H-indol-2-one,
- la N-(1,1-diméthyléthyl)-3-/2-/2-(1H-indol-4-yl) éthyl/phénoxy/propanamine, ainsi que leurs sels d'addition avec les acides minéraux ou organiques.

L'invention a également pour objet un procédé de préparation des dérivés, tels que définis par la formule (I) ci-dessus, ainsi que de leurs sels, caractérisé en ce que l'on fait réagir le 4-formyl indole de formule (II):

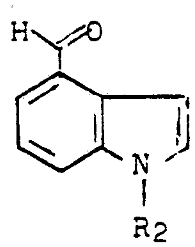

(II)

dans laquelle $R_2$ a la signification déjà indiquée, avec un halogénure d'hydroxybenzyl triphényl phosphonium de formule (III):

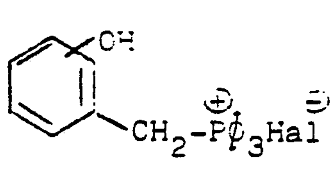

(III)

dans laquelle le radical hydroxy se trouve en position 2, 3 ou 4 et Hal représente un atome de chlore ou de brome, pour obtenir un dérivé de formule (IV):

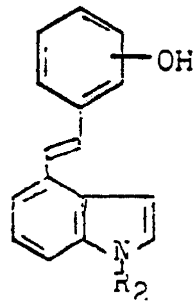

(IV)

dans laquelle $R_2$ a la signification déjà indiquée, que l'on fait réagir, ou bien avec l'halogénure de formule (V):

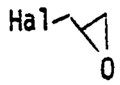

(V)

dans laquelle Hal a la signification déjà indiquée, pour obtenir un dérivé de formule (VI):

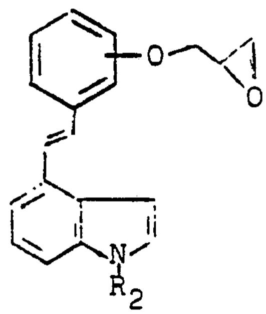

(VI)

dans laquelle $R_2$ a la signification déjà indiquée que l'on fait réagir avec l'amine primaire ou secondaire de formule (VII):

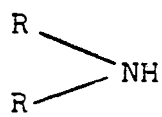

(VII)

dans laquelle R et $R_1$ ont la signification déjà indiquée, pour obtenir un produit de formule ($I_A$):

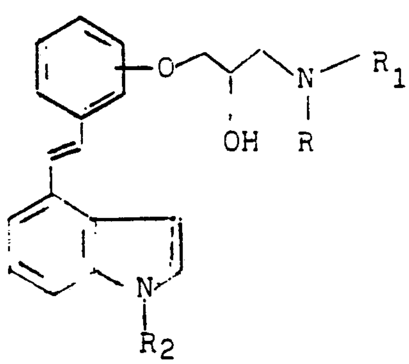

($I_A$)

dans laquelle R, $R_1$ et $R_2$ ont la signification déjà indiquée, que soit l'on isole et, si désiré, salifie, soit l'on soumet à une hydrogénation pour obtenir un produit de formule ($I_B$):

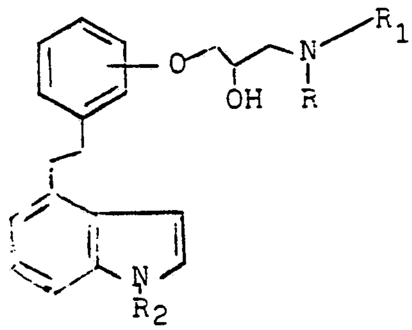

$(I_B)$

dans laquelle R, $R_1$ et $R_2$ ont la signification déjà indiquée, que, si désiré, l'on salifie,
ou bien l'on fait réagir ledit produit de formule (IV) avec l'halogénure de formule (V'):

$$Hal\text{-}(CH_2)_n\text{-}OR_3 \qquad (V')$$

dans laquelle Hal et n ont la signification déjà indiquée et $R_3$ représente un radical tosyle ou un atome d'hydrogène, pour obtenir un produit de formule (VI'):

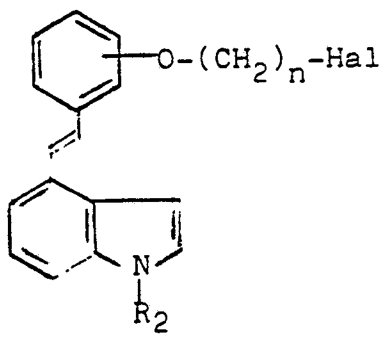

(VI')

dans laquelle n Hal et $R_2$ ont la signification déjà indiquée, que l'on fait réagir avec l'amine de formule (VII) définie ci-dessus, pour obtenir un produit de formule ($I'_A$):

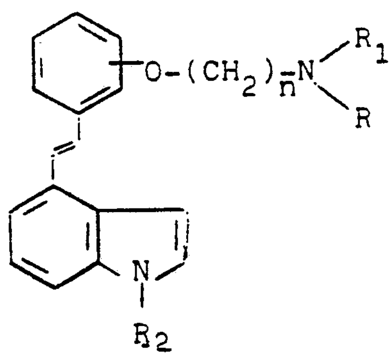

($I'_A$)

dans laquelle n, R, $R_1$ et $R_2$ ont la signification déjà indiquée, que soit l'on isole et, si désiré, salifie, soit l'on soumet à une hydrogénation pour obtenir un produit de formule ($I'_B$):

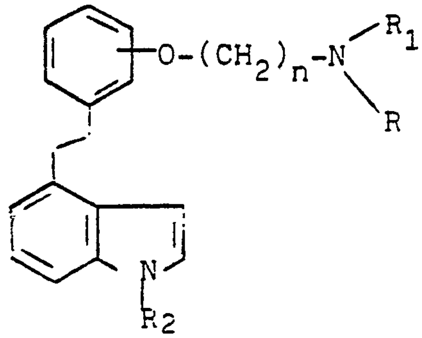

(I'B)

dans laquelle n, R, $R_1$ et $R_2$ ont la signification déjà indiquée, puis soumet, si désiré lesdits produits de formules ($I_A$), ($I_B$), ($I'_A$) et ($I'_B$) à l'action d'un agent d'halogénation pour obtenir un produit de formule (VIII):

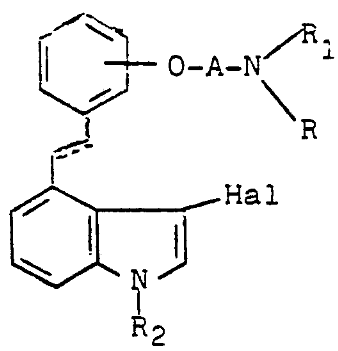

(VIII)

dans laquelle A, Hal, R, $R_1$, $R_2$ et le pointillé ont la signification déjà indiquée, que l'on soumet à une hydrolyse pour obtenir un produit de formule ($I_C$):

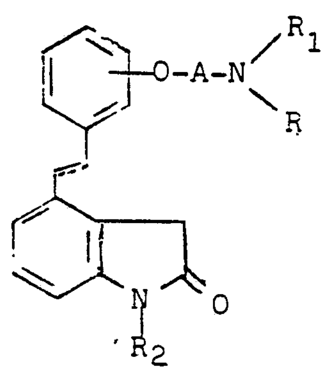

($I_C$)

dans laquelle A, R, $R_1$, $R_2$ et le pointillé ont la signification déjà indiquée, que l'on isole et, si désiré, salifie.

La réaction du 4-formyl indole avec le produit de formule (III) peut être effectuée directement dans un solvant ou un mélange de solvants tels que l'hexane ou le tétrahydrofuranne, mais de préférence, en présence d'une base organique telle que le butyl lithium ou d'un alcoolate alcalin ou encore d'un amidure alcalin tel que le diisopropyl amidure de lithium.

La réaction du produit de formule (IV) avec l'halogénure (V) peut être réalisée dans un solvant ou mélange de solvants inertes tels que le tétrahydrofuranne, le dioxanne, la diméthylformamide et notamment, l'acétone; elle est réalisée de préférence en présence d'un agent de condensation telle qu'une base comme la soude, la triéthylamine ou un carbonate alcalin comme le carbonate de potassium ou de sodium.

L'ouverture de l'époxyde par l'amine de formule (VII) peut être utilisé directement dans l'amine qui sert ainsi de solvant, soit dans un solvant tel qu'un alcool aliphatique comme l'éthanol.

La réaction du produit de formule (IV) avec l'halogénure de formule (V') est réalisée de préférence en présence de triphényl phosphine et d'azodicarboxylate de diéthyle dans le cas où $R_3$ représente un atome d'hydrogène. Dans le cas où $R_3$ représente un radical tosyle, la réaction a lieu de préférence en présence d'une base telle que la soude ou la potasse, ou telle qu'un alcoolate alcalin comme l'éthylate de sodium, ou en présence d'un hydrure alcalin comme l'hydrure de sodium.

L'hydrogénation des produits de formules ($I_A$) et ($I'_A$) peut être effectuée chimiquement par exemple par l'hydrazine, soit de manière catalytique et on utilise dans ce cas l'hydrogène en présence de platine, de palladium, ou de préférence de nickel de Raney.

L'halogénation des produits de formules ($I_A$), ($I'_A$), ($I_B$) et ($I'_B$) peut être réalisée, par exemple, à l'aide du complexe bromé de la pyridine de formule:

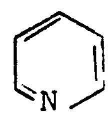 , $Br_2$, HBr

dans le cas de la bromation. Elle est réalisée avantageusement à l'aide d'un N-halo succinimide, de préférence le N-bromo ou le N-chloro succinimide; on opère dans le dioxanne ou de préférence dans l'acide acétique. Le produit de formule (VIII) obtenu est de préférence un produit chloré.

L'hydrolyse du produit de formule (VIII) est réalisée, de préférence à l'aide d'un acide minéral tel que l'acide phosphorique, l'acide sulfurique, ou de préférence l'acide chlorhydrique en solution aqueuse. Cette solution peut être utilisée concentrée, mais de préférence diluée par exemple en solution normale. On peut utiliser en outre un solvant tel qu'un alcool aliphatique comme l'éthanol.

Dans une variante, pour préparer le produit de formule ($I'_A$), on peut faire:

a) soit réagir un produit de formule (IV) avec un dihalogénure de formule (V''):

$$Hal\text{-}(CH_2)_n\text{-}Hal' \qquad (V'')$$

dans laquelle Hal a la signification déjà indiquée et Hal' a la signification de Hal, pour obtenir le produit de condensation halogéné repondant, que l'on fait réagir avec une amine de formule (VII):

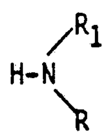 (VII)

dans laquelle R et $R_1$ ont la signification déjà indiquée, de manière à obtenir le produit de formule ($I'_A$) attendu,

b) soit réagir un produit de formule (IV) avec un halogénure de formule (V'',):

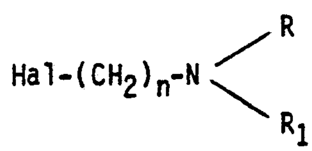 (V"')

dans laquelle Hal, R et $R_1$ ont la signification déjà indiquée, dans des conditions analogues à celles de la reaction avec l'halogénure de formule (V), pour obtenir le produit de formule ($I'_A$) attendu.

Dans une autre variante, le substituant $R_2$, lorsqu'il représente un radical alkyle, peut être fixé en dernière étape du procédé, par exemple par action d'un halogénure (chlorure, bromure ou iodure) d'alcoyle sur un produit de formule (I) dans laquelle $R_2$ représente un atome d'hydrogène, de préférence en présence d'un base comme ci-dessus.

Le produit de formule (II) est décrit notamment dans J. Org. Chem. (1980) 45 p. 3350 et suivantes. Les produits de formule (III) sont des produits connus; leur préparation est notamment decrite dans Tetrahedron 1981, Vol. 37, N° 16, p. 2867 et suivantes.

Les dérivés de formule (I) présentent un caractère basique. On peut avantageusement préparer les sels d'addition des dérivés de formule (I), en faisant réagir, en proportions sensiblement stoechiométriques, un acide minéral ou organique avec ledit dérivé de formule (I). Les sels peuvent être préparés sans isoler les bases correspondantes.

Les dérivés, objet de la présente invention, possèdent de très intéressantes propriétés pharmacologiques; ils sont doués notamment de propriétés bloquantes des canaux calcicosodiques lents et antihypertensives.

Certains dérivés possèdent en outre des propriétés α- et/ou β-bloquantes.

Ils sont également doués de propriétés anti-arythmiques. Ces propriétés sont illustrées plus loin dans la partie expérimentale. Ces propriétés justifient l'utilisation des dérivés de l'indol éthényl phénol, ainsi que de leurs sels, pharmaceutiquement acceptables à titre de médicaments.

La présente demande a ainsi également pour objet l'application, à titre de médicaments, des dérivés de l'indol éthényl phénol, tels que définis par la formule (I), ainsi que de leurs sels d'addition avec les acides pharmaceutiquement acceptables.

Parmi les médicaments, objet de l'invention, on retient de préférence, les médicaments caractérisés en ce qu'ils sont constitués par les nouveaux dérivés de l'indol éthényl phénol répondant à la formule (I), dans laquelle $R_2$ représente un atome d'hydrogène, A représente une chaîne

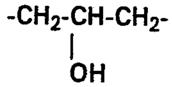

et R et $R_1$ représentent un atome d'hydrogène ou un radical alkyle, ainsi que par leurs sels d'addition avec les acides pharmaceutiquement acceptables.

Parmi les médicaments selon l'invention, on peut encore citer ceux caractérisés en ce qu'ils sont constitués par les nouveaux dérivés de formule (I), dans laquelle $R_2$ représente un atome d'hydrogène, A représente une chaîne $-(CH_2)_n-$, n étant défini comme précédemment et R et $R_1$ représentent un atome d'hydrogène ou un radical alkyle, ainsi que par leurs sels d'addition avec les acides pharmaceutiquement acceptables.

Parmi les médicaments selon l'invention, on peut encore citer ceux caractérisés en ce qu'ils sont constitués par les nouveaux dérivés de formule (I), dans laquelle la chaîne latérale

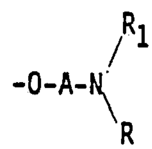

est fixée en position ortho, ainsi que par leurs sels d'addition avec les acides pharmaceutiquement acceptables.

Parmi les différents médicaments préférés ci-dessus, on retient notamment ceux constitués par les dérivés de formule (I) dans laquelle R représente un atome d'hydrogène, et a et b représentent ensemble une liaison carbone-carbone, ainsi que par leurs sels d'addition avec les acides pharmaceutiquement acceptables et tout particulièrement, les dérivés dont les noms suivent:

- le 1-/(1,1-diméthyl éthyl amino/-3-/(4-/2-(1H-indol-4-yl) éthényl/phénoxy/2-propanol,
- le 1-/(1,1-diméthyl éthyl)amino/-3-/2/2-(1H-indol-4-yl)éthyl/phénoxy/2-propanol,
- la 1,3-dihydro 4-/2-/2-/3-/(1,1-diméthyl éthyl)amino/2-hydroxy propoxy/phényl/éthényl/2H-indol-2-one,
- la N-(1,1-diméthyléthyl)-3-/2-/(1H-indol-4-yl) éthyl/phénoxy/propanamine, ainsi que leurs sels d'addition avec les acides pharmaceutiquement acceptables.

Les médicaments selon l'invention trouvent leur emploi, par exemple, dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé ou atteint d'artériosclérose et dans le traitement de l'hypertension d'origine rénale. Ils sont également utilisables dans le traitement de l'insuffisance cardiaque, de l'angor sous toutes ses formes ainsi que dans le traitement des arythmies.

La dose usuelle, variable selon le dérivé utilisé, le sujet et l'affection en cause peut être par exemple de 10 mg à 500 mg par jour. Par voie orale, chez l'homme, le dérivé de l'exemple 3 peut être administré à la dose quotidienne de 10 mg à 100 mg, par exemple pour le traitement de l'angor, soit environ de 0,15 mg à 1,5 mg par kilogramme de poids corporel.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humains, comme par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits industriels nouveaux utiles notamment pour la préparation des dérivés répondant à la formule (I), à savoir les produits de formule (IV):

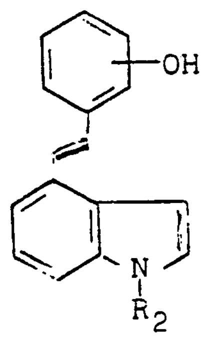

(IV)

dans laquelle $R_2$ a la signification déjà indiquée.

Les exemples suivants illustrent l'invention sans toutefois la limiter.

**Exemple 1:** 1-/2-/2 (1H-indol-4-yl) éthényl/ phénoxy/-3-/(1-méthyl éthyl) amino/-2-propanol et son chlorhydrate.

Stade A: 2-/(1H-indol-4-yl) éthényl/ phénol.

On met en suspension sous atmosphère inerte 31 g de bromure d'ortho hydroxybenzyl triphényl phosphonium dans 350 $cm^3$ de tétrahydrofuranne, ajoute en 1 heure 86 $cm^3$ d'une solution 1,6 M de butyl lithium dans l'hexane, laisse 30 mn sous agitation, ajoute en 30 mn une solution de 5 g de 4-formyl indole dans 150 $cm^3$ de tétrahydrofuranne et laisse sous agitation pendant 24 heures. On dilue alors par 500 $cm^3$ d'eau, ajoute du carbonate de potassium à saturation, extrait à l'acétate d'éthyle, isole par chromatographie sur silice (éluant: chlorure de méthylène) les fractions de Rf=0,20, empâte les cristaux obtenus au chlorure de méthylène, filtre, sèche sous pression réduite et obtient 7,4 g du produit attendu F 140°C, recristallisé dans le chlorure de méthylène F 142°C.

Stade B: 4-/2-/2-/(2-oxiranyl)méthoxy/phényl/éthényl/1H-indol.

On porte au reflux pendant 24 heures, sous agitation et atmosphère inerte 7,25 g du produit obtenu ci-dessus avec 150 $cm^3$ d'acétone, 4,4 g de carbonate de potassium et 3 $cm^3$ d'épichlorhydrine, filtre, élimine le solvant à 50°C sous pression réduite, sépare par chromatographie sur silice (éluant: chlorure de méthylène) les fractions de Rf=0,40 et obtient 5,8 g d'huile jaune après élimination des solvants.

Stade C: 1-/2-/2-(1H-indol-4-yl)éthényl/phénoxy/-3-/(1-méthyl éthyl) amino/-2-propanol et son chlorhydrate.

On agite sous atmosphère inerte pendant 20 heures 4,8 g de l'huile obtenue au stade B dans 5,7 $cm^3$ d'isopropylamine, évapore le solvant à 50°C sous pression réduite, empâte les cristaux obtenus à l'éther de pétrole (Eb: 60.- 80°C), filtre, sèche sous pression réduite, purifie par chromatographie sur silice (éluant: acétate d'éthyle - triéthylamine 9-1) et obtient 5 g de la base du produit attendu. F≅110°C.

Formation du chlorhydrate:

On dissout la base ci-dessus dans 100° $cm^3$ d'acétate d'éthyle, ajoute un excès de solution saturée d'acide chlorhydrique dans l'acétate d'éthyle, concentre, glace, filtre et sèche sous pression réduite le produit que l'on recristallise dans 200 $cm^3$ d'isopropanol et 400 $cm^3$ de méthanol au reflux et obtient 4 g du produit attendu. F≅254°C.

Analyse: pour $C_{22}H_{26}N_2O_2$, HCl = 386,925

| | | | | |
|---|---|---|---|---|
| Calculé | C % 68,29 | H % 7,03 | N % 7,27 | Cl % 9,16 |
| Trouvé | 68,2 | 7,1 | 7,3 | 9,3. |

**Exemple 2:** 1-/(1,1-diméthyl éthyl)amino/-3-/2-/2-(1H-indol-4-yl)éthényl/phénoxy/-2-propanol et son chlorhydrate.

En opérant selon un procédé analogue à celui décrit ci-dessus à l'exemple 1 en remplaçant l'isopropylamine par la tert-butylamine, on obtient le produit attendu et son chlorhydrate F≅230°C.

Analyse: $C_{23}H_{29}N_2ClO_2$ = 400,952.

| | | | | |
|---|---|---|---|---|
| Calculé | C % 68,90 | H % 7,29 | N % 6,99 | Cl % 8,84 |
| Trouvé | 68,9 | 7,4 | 6,8 | 8,8. |

**Exemple 3**: 1-/(1,1-diméthyl éthyl)amino/-3-/4-/2-(1H-indol-4-yl) éthényl/phénoxy/-2-propanol et son chlorhydrate.

Stade A: 4-/(1H-indol-4-yl) éthényl/ phénol.

On met en suspension sous atmosphère inerte 31 g de bromure de parhydroxybenzyl triphényl phosphonium dans 350 cm³ de tétrahydrofuranne, ajoute en 1 heure 86 cm³, d'une solution 1,6 M de butyllithium dans l'hexane, laisse 30 mn sous agitation, ajoute en 30 mn une solution de 5 g de 4-formyl indole dans 100 cm³ de tétrahydrofuranne et laisse sous agitation pendant 24 heures. On dilue alors par 500 cm³ d'eau, extrait à l'acétate d'éthyle, isole par chromatographie sur silice (éluant: chlorure de méthylène) les fractions de Rf=0,15, empâte les cristaux obtenus à l'éther de pétrole (Eb=60°- 80°C), filtre, sèche sous pression réduite et obtient 7 du produit attendu F≅145°C.

Stade B: 4-/2-/4-/(2-oxiranyl) méthoxy/ phényl/ éthényl/ 1H-indole.

On porte au reflux pendant 24 heures sous agitation et atmosphère inerte 5,8 g du produit obtenu ci-dessus avec 120 cm³ d'acétone 3,6 g de carbonate de potassium et 9,6 cm³ d'épichlorhydrine, ajoute 9,6 cm³ d'épichlorhydrine et poursuit le reflux pendant 5 heures, filtre, élimine le solvant à 60°C sous pression réduite, sépare par chromatographie sur silice (éluant: chlorure de méthylène) les fractions de Rf=0,35 et obtient 5,25 g du produit attendu F≅128°C.

Stade C: 1-/(1,1-diméthyléthyl) amino/-3-/4-/2-(1H-indol-4-yl) éthényl/phénoxy/-2-propanol et son chlorhydrate.

On porte au reflux sous agitation et atmosphère inerte pendant 1 heure 7,5 g du produit tel qu'obtenu au stade précédent avec 27 cm³ de tert-butylamine dans 100 cm³ d'éthanol, évapore le solvant à 50°C sous pression réduite, purifie par chromatographie sur silice (éluant: chloroforme - acétone - triéthylamine 6-3-1) et obtient 9,4 g de la base du produit attendu.

Formation du chlorhydrate.

On dissout 9,4 g de base ci-dessus dans 400 cm³ d'isopropanol, ajoute un excès de solution saturée d'acide chlorhydrique dans l'acétate d'éthyle, concentre, glace, filtre et sèche sous pression réduite 9,6 g de produit brut. On recristallise 5 g de produit brut dans 300 cm³ d'isopropanol et 200 cm³ de méthanol au reflux et obtient 4,5 g du produit attendu. F≅256°C.

Analyse: pour $C_{23}H_{28}N_2O_2$, HCl = 400,952

| | | | | |
|---|---|---|---|---|
| Calculé | C % 68,90 | H % 7,29 | N % 6,99 | Cl % 8,84 |
| Trouvé | 68,9 | 7,4 | 7,0 | 8,9 |

**Exemple 4**: 1-/4-/2-(1H-indol-4-yl) éthényl/ phénoxy/-3-/(1-méthyléthyl) amino/-2-propanol et son chlorhydrate.

En opérant selon un procédé analogue à celui décrit ci-dessus à l'exemple 1 en remplaçant l'orthohydroxy benzyl triphényl phosphonium par le parahydroxy benzyl triphényl phosphonium, on obtient le produit attendu.
Chlorhydrate: F≅250°C.
Analyse: $C_{22}H_{27}N_2ClO_2$ = 386,925

| | | | | |
|---|---|---|---|---|
| Calculé | C % 68,29 | H % 7,03 | N % 7,27 | Cl % 9,16 |
| Trouvé | 68,3 | 7,1 | 7,1 | 9,2 |

**Exemple 5**: 1,3-dihydro 4-/2-/4-/3-/(1,1-diméthyl éthyl) amino/ 2-hydroxy propoxy/ phényl/ éthényl/ 2H-indol-2-one et son chlorhydrate.

Halogénation

On agite sous atmosphère inerte pendant 2 heures 4,24 g de la base du produit de l'exemple 3 dans 60 cm³ d'acide acétique avec 1,7 g de N-chlorosuccinimide. On dilue à l'eau, alcalinise à l'aide d'ammoniaque, sature

de carbonate de potassium, extrait à l'acétate d'éthyle, isole par chromatographie sur silice (éluant: acétate d'éthyle-triéthylamine 9-1) les fractions de Rf=0,20 et obtient 3,4 g de produit chloré.

Hydrolyse

On chauffe au reflux pendant 2 heures sous agitation et atmosphère inerte le produit chloré ci-dessus dans 50 $cm^3$ d'éthanol et 100 $cm^3$ d'acide chlorhydrique N, refroidit, dilue par 200 $cm^3$ d'eau, glace, filtre, lave avec de l'éthanol, séche à 80°C sous pression réduite et obtient 2,1 g du chlorhydrate du produit attendu F≅280°C.

Les eaux-mères sont alcalinisées par addition de soude et extraites à l'acétate-d'éthyle; on purifie parchromatographie sur silice (éluant acétate d'éthyle-triéthylamine 9-1) et obtient 500 mg de la base du produit attendu F≅110°C.

Analyse pour $C_{23}H_{29}N_2ClO_3$ = 416,951

| | | | | |
|---|---|---|---|---|
| Calculé | C % 66,26 | H % 7,01 | N % 6,72 | Cl % 8,50 |
| Trouvé | 66,0 | 7,2 | 6,6 | 8,6 |

**Exemple 6**: N-(1,1-diméthyl éthyl)-3-/2-/2-(1H-indol-4-yl) éthényl/ phénoxy/ propanamine et son succinate.

Stade A: 4-/2-(2-(3-chloro propoxy) phényl/ éthényl/ 1H-indol.

On agite pendant 5 heures sous atmosphère inerte 235 mg de-2-/(1H-indol-4-yl) éthényl/ phénol dans 20 $cm^3$ de tétrahydrofuranne, avec 0,15 $cm^3$ d'azodicarboxylate de diéthyle, 0,1 $cm^3$ de 3-chloro 1-propanol et 262 mg de triphényl phoshine et ajoute 0,1 $cm^3$ de 3-chloro-1-propanol, 0,15 $cm^3$ d'azodicarboxylate de diéthyle et 262 mg de triphényl phosphine après 15 heures d'agitation à température ambiante, on concentre à sec et purifie par chromatographie sur silice (éluant: benzène). On obtient 310 mg du produit attendu.

Stade B: N-(1,1-diméthyl éthyl)-3-/2-/2-(1H-indol-4-yl) éthényl/ phénoxy/ propanamine et son succinate.

On met en solution 250 mg du produit ci-dessus dans 2 $cm^3$ de diméthyl formamide, ajoute 2 $cm^3$ de tertbutylamine et 110 mg de carbonate de potassium, chauffe 3 heures à 120°C sous 5 kg de pression, dilue à l'eau, extrait à l'acétate d'éthyle, lave à l'eau et obtient 295 mg d'une résine que l'on purifie par chromatographie sur silice (éluant acétate d'éthyle-triéthylamine 9-1) et obtient 235 mg de la base du produit attendu.

Formation du succinate

On dissout 8,9 g de base telle que préparée ci-dessus dans 300 $cm^3$ d'isopropanol, ajoute 3 g d'acide succinique, chauffe au reflux, concentre à 200 $cm^3$, glace, filtre, sèche sous pression réduite et obtient 8,7 g du succinate attendu F≅200°C.

**Exemple 7**: 1-/(1,1-dimethyl éthyl) amino/-3-/3-/2-(1H-indol-4-yl) éthényl/ phénoxy/-2-propanol et son chlorhydrate.

En opérant selon un procédé analogue à celui décrit à l'exemple 1, à partir de méta hydroxy benzyl triphényl phosphonium, on obtient le chlorhydrate attendu F≅184°C aprés recristallisation dans l'acétonitrile.

Analyse $C_{22}H_{27}N_2ClO_2$ = 386,925

| | | | | |
|---|---|---|---|---|
| Calculé | C % 68,29 | H % 7,03 | N % 7,27 | Cl % 9,16 |
| Trouvé | 68,0 | 7,0 | 7,1 | 9,0 |

**Exemple 8**: 1-/(1,1-diméthyl éthyl) amino/-3-/2-/2-(1H-indol-4-yl) éthyl/ phénoxy/-2-propanol.

On hydrogène pendant 1 heure 365 mg de base de l'exemple 2 dans 20 $cm^3$ d'éthanol, en présence de 100 mg de palladium à 10 % sur charbon, filtre, élimine le solvant à 50°C sous pression réduite et obtient 325 mg du produit attendu.

RMN (250 MHz) $CDCl_3$

| | | |
|---|---|---|
| H terbutyle | | 275 Hz |
| $CH_2$ en α de l'azote | 685 à | 732 Hz |
| $CH_2$ de l'éthyl | 752 à | 802 Hz |
| $CH_2$ en α de l'oxygène | 977 à | 1025 Hz |

Formation du chlorhydrate:

On dissout 4,5 g de produit obtenu comme ci-dessus dans 200 $cm^3$ d'isopropanol et ajoute une solution saturée d'acide chlorhydrique dans l'isopropanol, on concentre sous pression réduite jusqu'à l'obtention d'un volume de 100 $cm^3$, glace, essore et sèche à 60°C sous pression réduite. On obtient 4,7 g de produit attendu. F=105°C.

**Exemple 9**: 1,3-dihydro 4-/2-/2-/3-/(1,1-diméthyléthyl)amino/2-hydroxy propoxy/phényl/éthényl/2H-indol-2-one et son chlorhydrate.

Stade A: Halogénation.

On opère comme à l'exemple 5 à partir de 10 g de la base obtenue à l'exemple 2 et obtient 6,9 g de dérivé chloré.

Stade B: 1,3-dihydro 4-/2-/2-/3-/(1,1-diméthyléthyl)amino/2-hydroxy propoxy/phényl/éthényl/2H-indol-2-one et son chlorhydrate.

On chauffe 2 heures au reflux 6,9 g du produit obtenu au stade A, 100 $cm^3$ d'éthanol et 200 $cm^3$ d'acide chlorhydrique N. On dilue avec 100 $cm^3$ d'eau glacée, alcalinise par addition de lessive de soude et extrait à l'acétate d'éthyle. On chromatographie sur silice (éluant: chloroforme - acétone - triéthylamine 6-3-1) et récupère 4,3 g de produit attendu. F=112°C.

Formation du chlorhydrate.

On dissout la base obtenue dans 200 $cm^3$ d'isopropanol, ajoute une solution saturée d'acide chlorhydrique dans l'isopropanol, chauffe 30 minutes au reflux, concentre jusqu'à l'obtention d'un volume de 100 $cm^3$, glace, essore et sèche à 80°C sous pression réduite. On obtient 3,65 g de produit attendu. F $\geqslant$ 260°C.

Analyse pour $C_{23}H_{28}N_2O_3$, HCl = 416,951.

| | | | | |
|---|---|---|---|---|
| Calculé | C % 66,26 | H % 7,01 | N % 6,72 | Cl % 8,50 |
| Trouvé | 66,3 | 7,1 | 6,8 | 8,7. |

**Exemple 10**: 1-/(1,1-diméthyléthyl)amino/3-/2-/2-(1-méthyl 1H-indol-4-yl)éthényl/phénoxy/2-propanol (trans E) et son oxalate neutre.

On chauffe 30 minutes à 40°- 50°C la suspension comprenant 9,2 g de la base obtenue à l'exemple 2, 100 $cm^3$ de diméthylformamide et 1,2 g d'hydrure de sodium.

On refroidit à 0°- 5°C et ajoute 1,7 $cm^3$ d'iodure de méthyle puis chauffe de nouveau 3 heures à 40°C. On laisse revenir à température ambiante, dilue avec 200 $cm^3$ d'eau, extrait à l'acétate d'éthyle, lave à l'eau, sèche et élimine le solvant à 50°C sous pression réduite. On chromatographie sur silice (éluant: chloroforme - acétone - triéthylamine 6-3-1) et obtient 5 g de produit attendu.

Formation de l'oxalate neutre.

On dissout 5 g de la base obtenue ci-dessus dans 200 $cm^3$ d'isopropanol et ajoute 1,7 g d'acide oxalique, puis 500 $cm^3$ de méthanol et chauffe au reflux jusqu'à dissolution totale. On concentre jusqu'à l'obtention d'un volume de 100 $cm^3$, glace, essore et sèche à 80°C sous pression réduite. On recristallise le produit obtenu dans un mélange d'éthanol et de méthanol et récupère 3,9 g d'oxalate. F=225°C.

**Exemple 11**: N-(1,1-diméthyléthyl)2-/2-/2-(1H-indol-4-yl)éthényl/phénoxy/éthanamine (trans E) et son tartrate acide.

Stade A: 4-/2-/2-/(2-chloro)éthoxy/phényl/éthényl/1H-indole (trans E).

On chauffe 6 heures au reflux 4 g de 2-/(1H-indol-4-yl)éthényl phénol obtenu comme au stade A de l'exemple 1 dans 100 $cm^3$ de soude 2 N après avoir ajouté 6 g de tosylate de 2-chloroéthanol. On laisse 16 heures à température ambiante, dilue à l'eau et extrait à l'acétate d'éthyle, élimine le solvant sous pression réduite. Après chromatographie sur silice (éluant: benzène), on récupère 2 g de produit attendu.

Stade B: N-(1,1-diméthyléthyl)2-/2-/2-(1H-indol-4-yl)éthényl/phénoxy/éthanamine (trans E) et son tartrate.

On chauffe 4 heures à 120°C, 1,65 g de produit obtenu au stade A précédent, 20 $cm^3$ de diméthylformamide, 15 $cm^3$ de terbutylamine et 770 mg de carbonate de potassium.

On dilue le mélange réactionnel avec 100 $cm^3$ d'eau, extrait à l'acétate d'éthyle, lave à l'eau, sèche, concentre sous pression réduite à 50°C. Après chromatographie sur silice (éluant: acétate d'éthyle - triéthylamine 9-1), on recueille 1,7 g de produit attendu.

Formation du tartrate acide.

On dissout 1,9 g de base préparée comme ci-dessus dans 200 $cm^3$ d'isopropanol et 100 $cm^3$ de méthanol, ajoute 850 mg d'acide DL tartrique et chauffe 30 minutes au reflux, filtre à chaud, concentre, glace et sèche sous pression réduite à 80°C. On obtient 2,55 g de produit attendu. F=180°- 182°C.

**Exemple 12**: Succinate neutre de N-(1,1-diméthyléthyl)3-/2-/2-(1H-indol-4-yl)éthyl/phénoxy/propanamine.

On hydrogène pendant 30 minutes 2,5 g du succinate de l'exemple 6 dans 500 $cm^3$ de méthanol en présence

de 850 mg de palladium à 10 % sur charbon, filtre, concentre sous pression réduite à 50° C jusqu'à l'obtention d'un volume de 50 $cm^3$, glace, filtre, séche sous pression réduite et obtient 2,25 g de produit attendu. F = 190° C.

**Exemple 13**: N-/2/2-/2-/(1H-indol-4-yl)éthyl/phénoxy/éthyl/2-méthyl 2-propanamine et son tartrate acide.

On opère comme à l'exemple 8 à partir de 550 mg de produit obtenu comme au stade 8 de l'exemple 11 et obtient 643 mg de produit attendu que l'on purifie par chromatographie sur silice (éluant: cyclohexane - chloroforme triéthylamine 6-3-1) et recueille 510 mg de produit pur.

Formation du tartrate acide.

On dissout 1,7 g de base préparée comme au stade A dans 200 $cm^3$ d'isopropanol, ajoute 760 mg d'acide DL tartrique, chauffe 15 minutes au reflux, filtre, concentre jusqu'à un volume de 100 $cm^3$, glace, essore et sèche à 80° C sous pression réduite. On obtient 1,75 g de produit attendu. F = 158° C.

Analyse: $C_{22}H_{28}N_2O$, $C_4H_6O_4$ = 486,57

| | | | |
|---|---|---|---|
| Calculé: | C % 64,18 | H % 7,04 | N % 5,76 |
| Trouvé: | 63,9 | 7,3 | 5,8 |

**Exemple 14**: N-/2-/2-/2-(1H-indol-4-yl)éthényl/phénoxy/éthyl/benzène éthanamine (E) et son fumarate neutre.

On ajoute 8,8 $cm^3$ de 2-phényléthylamine à une solution de 3,48 g de produit obtenu comme au stade A de l'exemple 11 dans 25 $cm^3$ d'éthanol et chauffe 28 heures au reflux. On refroidit le mélange réactionnel, le verse dans l'eau et extrait à l'éther. On lave à l'eau, puis avec une solution aqueuse de chlorure de sodium, sèche et concentre à sec.

On chromatographie le résidu sur silice (éluant: cyclohexane - chloroforme - triéthylamine 6-3-1) et obtient 3,54 g de produit attendu.

Préparation du fumarate neutre.

On ajoute une solution de 0,24 g d'acide fumarique dans 15 $cm^3$ d'éthanol à 1,61 g de base obtenue ci-dessus en solution dans 32 $cm^3$ d'éthanol et maintient sous agitation pendant 1 heure 30. On filtre, rince à l'éthanol, sèche et obtient 1,41 g de fumarate neutre que l'on recristallise dans le méthanol et recueille 0,71 9 de produit pur. F = 184-185° C.

Analyse: $C_{26}H_{26}N_2O$, 1/2 $C_4H_4O_4$ = 881,085

| | | | |
|---|---|---|---|
| Calculé: | C% 76,33 | H% 6,4 | N% 6,35 |
| Trouvé: | 76,0 | 6,5 | 6,4. |

**Exemple 15**: N-/2-/2-/2-(1H-indol-4-yl)éthyl/phénoxy/éthyl benzène éthanamine et son chlorhydrate.

On hydrogène 3,54 g de la base obtenue à l'exemple 14 dans 110 $cm^3$ d'éthanol en présence de 1,18 g de palladium à 10 % sur charbon, filtre, élimine le solvant sous pression réduite et obtient 3,32 g de produit attendu.

Préparation du Chlorhydrate.

On dissout 3,2 g de la base obtenue ci-dessus dans 65 $cm^3$ d'isopropanol, ajoute un excès de solution saturée d'acide chlorhydrique dans l'acétate d'éthyle, amorce la cristallisation, glace, filtre et sèche sous pression réduite. On obtient 2,6 g de produit attendu que l'on recristallise dans l'isopropanol. On récupére 1,64 g de produit pur. F = 177 - 178° C.

Analyse: $C_{26}H_{28}N_2O$, HCl = 420,991.

| | | | | |
|---|---|---|---|---|
| Calculé: | C % 74,17 | H % 6,94 | Cl % 8,42 | N % 6,65 |
| Trouvé: | 73,9 | 7,0 | 8,6 | 6,6. |

Selon le procédé de l'invention, on peut encore notamment préparer:
- la N-/2-/2-/2-(1H-indol-4-yl)éthyl/phénoxy/éthyl/diméthylamine,
- la N-/2-/2-/2-(1H-indol-4-yl)éthényl/phénoxy/éthyl/diméthylamine,
- la N-/2-/2-/2-(1H-indol-4-yl)éthyl/phénoxy/éthyl/diéthylamine,
- la N-/2-/2-/2-(1H-indol-4-yl)éthényl/phénoxy/éthyl/diéthylamine,
- la N-/2-/2-/2-(1H-indol-4-yl)éthyl/phénoxy/éthyl/pipéridine et
- la N-/2-/2-/2-(1H-indol-4-yl)éthényl/phénoxy/éthyl pipéridine et leurs sels.

**Exemple 16**:

On a préparé des comprimés répondant à la formule:

- produit de l'exemple 3 (chlorhydrate) 20 mg
- excipient q. s. pour un comprimé terminé à 100 mg

(Détail de l'excipient: lactose, amidon, talc, stéarate de magnésium).

**Exemple 17**:

On a préparé des comprimés répondant à la formule:

- produit de l'exemple 2 (chlorhydrate) 50 mg
- excipient q. s. pour un comprimé terminé à 100 mg

(Détail de l'excipient: lactose, amidon, talc, stéarate de magnésium).

**Etude pharmacologique**

1) Test d'activité anticalcique in vitro

Des artères caudales de rat découpées en spirale sont reliées à des capteurs de tension et sont maintenues dans des cuves de 25 ul de tampon Krebs-bicarbonate de sodium (NaCl : 120,8 mM, KCl : 5,g mM, $MgCl_2$ : 1,2 mM, $NaH_2PO_4$ : 1,2 mM, $NaHCO_3$ : 15,5 mM, glucose : 12,5 mM) à 37°C gazées avec un mélange $O_2$ : 95 % - $CO_2$: 5 %.

Les préparations sont dépolarisées par une solution tampon à concentration 100 mM en ions K+ (NaCl : 26,7 mM, KCl : 100 mM, $MgCl_2$ 1,2 mM, $NaH_2PO_4$: 1,2 mM, $NaHCO_3$: 15,5 mM, glucose: 12,6 mM).

On ajoute, sous un volume de 250 ul, du chlorure de calcium, de manière à obtenir une gamme de concentrations croissantes en ions $Ca^{2+}$ allant de 0,1 à 3,0 mM ; on enregistre les contractions des artères et établit ainsi une gamme témoin. On répète l'opération avec la gamme d'ions $Ca^{+2}$ toutes les 15 minutes et la préparation est lavée quatre fois après chaque gamme.

Lorsque l'on obtient une réponse stable, l'on effectue l'opération avec les gammes d'ions $Ca^{2+}$ en présence de différentes concentrations du produit à tester, jusqu'à ce qu'une réponse stable soit obtenue.

Les contractions des artères dépendent de l'entrée des ions $Ca^{2+}$ dans les cellules des muscles lisses et sont provoquées par la dépolarisation du muscle lisse par les ions K+ et par l'action de la noradrénaline libérée au niveau présynaptique. En recommençant l'opération avec des artères dénervées par action de 6-OH dopamine, on supprime l'action propre due à la noradrénaline.

Les résultats sont exprimés en Cl 50 (concentration inhibitrice 50) concentration du produit testé qui inhibe de 50 % la concentration due aux ions K+.

On constate d'après les résultats figurant sur le tableau ci-après que les produits de la présente demande possèdent une forte activité anticalcique.

| Produit de l'exemple | Cl 50 en uM |
|---|---|
| 1 | 4,0 |
| 2 | 1,2 |
| 3 | 0,6 |
| 4 | 3,5 |
| 5 | 2,5 |
| 6 | 2 |
| 8 | 1,5 |
| 9 | 1,4 |
| 12 | 0,3 |

2) Action antiarythmique chez le rat

On trachéotomise des rats mâles pesant 300 - 350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse.

Cinq minutes après l'administration du produit, on perfuse la veine jugulaire des rats avec 10 ug/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains produits de la présente demande sont doués de bonnes propriétés antiarythmiques.

| Produit de l'exemle | Dose | Pourcentage d'allongement du temps |
|---|---|---|
| 2 | 10 mg/kg | 110 % |
| | 5 mg/kg | 30 % |
| 6 | 5 mg/kg | 49 % |
| 8 | 5 mg/kg | 162 % |
| 9 | 5 mg/kg | 167 % |
| 11 | 5 mg/kg | 41 % |

3) Détermination de l'activité hypotensive

L'activité hypotensive a été étudiée sur des rats mâles de souche WISTAR pesant 300 g environ et anesthésiés au pentobarbital sodique (50 mg/kg par voie intraveineuse).

Le produit testé a été administré par voie intraveineuse dans la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témoin initiale.

| Produit de l'exemple | Dose mg/kg | Variation % de la pression artérielle | | | |
|---|---|---|---|---|---|
| | | 1 mn après administration | 5 mn après administration | 10 mn après administration | 30 mn après administration |
| 1 | 1 | - 11 | - 11 | - 12 | - 13 |
| 2 | 1 | - 17 | - 10 | - 10 | - 14 |

4) Affinité pour les récepteurs $\alpha_2$ adrénergiques

On homogénéise dans 90 ml de sucrose 0,32 M, 10 cortex prélevés sur des cerveaux de rats mâles pesant 150 g en moyenne. Après centrifugation à 1 000 g du mélange homogénéisé pendant 10 minutes à 0°C, le surnageant est centrifugé à 30 000 g pendant 10 minutes à 0° +4°C. Le culot est mis en suspension dans 240 ml de tampon TrisHCl 50 mM pH 7,7 et centrifugé à 30 000 g pendant 15 minutes à 0° +4°C. Le nouveau culot obtenu est mis en suspension dans 480 ml de tampon $NaKPO_4$ pH 7,4 50 mM.

On fait ensuite incuber pendant 45 minutes à 25°C, 2 ml de suspension en présence de $^3H$ rauwolscine à la concentration 0,15 nM:

i) seule,

ii) avec des concentrations croissantes du produit à tester ou,

iii) pour déterminer la fixation nonspécifique,

avec de la phentolamine non radioactive à la concentration $10^{-5}$ M.

Les suspensions incubées sont filtrées sur Whatman GF/C et les filtres sont lavés par trois fois 5 ml de tampon $NaKPO_4$ pH 7,4 à 0°C.

La radioactivité des filtres est mesurée par scintillation liquide.

L'affinité du produit testé pour les récepteurs α 2 adrénergiques est donnée relativement à la phentolamine comme produit de référence. CD = concentration de phentolamine inhibant 50 % de la fixation spécifique de la $^3H$ rauwolscine;

CX = concentration du produit à tester inhibant 50 % de la fixation spécifique de la $^3H$ rauwolscine.

L'affinité relative est donnée par la relation: $ARL = 100 \frac{CD}{CX}$

On a obtenu les résultats suivants:

| Produit de l'exemple | ARL en % |
|---|---|
| 1 | 0,1 |
| 2 | 0,25 |
| 3 | 0,7 |
| 4 | 0,0 |

Ces résultats montrent que les produits de la présente demande présentent une affinité notable pour les récepteurs $\alpha_2$ adrénergiques.

5) Affinité pour les récepteurs $\beta_1$ adrénergiques

On homogénéise dans 90 ml de sucrose 0,32 M, 10 cortex prélevés sur des cerveaux de rats mâles pesant 150 g en moyenne. Après centrifugation à 1 000 g du mélange homogénéisé pendant 20 minutes à 0°C, le surnageant est centrifugé à 30 000 g pendant 15 minutes à 0° +4°C. Le culot est mis en suspension dans 120 ml

de tampon TrisHCl 50 mM pH 7,7 et centrifugé à 30 000 g pendant 15 minutes à 0° +4° C. Le nouveau culot obtenu est mis en suspension dans 480 ml de tampon Krebs Tris HCl 7,7 50 mM.

On fait ensuite incuber pendant 10 minutes à 37° C 2 ml de suspension en présence de $^3H$ dihydroalprénolol à la concentration $10^{-9}$ M:

i) seule,

ii) avec des concentrations croissantes du produit à tester ou,

iii) pour déterminer la fixation non spécifique, avec du propranolol non radioactif à la concentration $10^{-5}$ M.

Les suspensions incubées sont filtrées sur Whatman GF/C et les filtres sont lavés par trois fois 5 ml de tampon Krebs Tris HCl pH 7,7 à 0° C.

La radioactivité des filtres est mesurée par scintillation liquide.

L'affinité du produit testé pour les récepteurs adrénergiques est donnée relativement au propranolol comme produit de référence. CD = concentration du propranolol inhibant 50 % de la fixation spécifique du $^3H$ dihydroalprénolol;

CX = concentration du produit à tester inhibant 50 % de la fixation spécifique du $^3H$ dihydroalprénolol.

L'affinité relative est donnée par la relation: $ARL = 100 \frac{CD}{CX}$.

On a obtenu les résultats suivants:

| Produit de l'exemple | ARL en % |
|---|---|
| 1 | 92,5 |
| 2 | 198 |
| 3 | 0,8 |
| 4 | 1,0 |

Ces résultats montrent que les produits de la présente demande présentent une affinité notable pour les récepteurs $\beta_1$ adrénergiques.

6) Affinité pour les récepteurs $\beta_2$ adrénergiques

On homogénéise dans 90 ml de sucrose 0,32 M, les cervelets prélevés sur des cerveaux de rats mâles pesant 150 g en moyenne. Après centrifugation à 1 000 g du mélange homogénéisé pendant 20 minutes à 0° C, le surnageant est centrifugé à 30 000 g pendant 15 minutes à 0° +4° C. Le culot est mis en suspension dans 120 ml de tampon TrisHCl 50 mM pH 7,7 et centrifuge a 30 000 g pendant 15 minutes à 0° +4° C. Le nouveau culot obtenu est mis en suspension dans 480 ml de tampon Krebs Tris HCl 7,7.

On fait ensuite incuber pendant 10 minutes à 37° C 2 ml de suspension en présence de $^3H$ dihydroalprénolol à la concentration $10^{-9}$ M:

i) seule,

ii) avec des concentrations croissantes du produit à tester ou,

iii) pour déterminer la fixation nonspécifique, avec du propranolol non radioactif à la concentration $10^{-5}$ M.

Les suspensions incubées sont filtrées sur Whatman GF/C et les filtres sont lavés par trois fois 5 ml de tampon Krebs Tris HCl pH 7,7 à 0° C.

La radioactivité des filtres est mesurée par scintillation liquide.

L'affinité du produit testé pour les récepteurs $\beta_2$ adrénergiques est donnée relativement au propranolol comme produit de référence.

CD = concentration du propranolol inhibant 50 % de la fixation spécifique du $^3H$ dihydroalprénolol;

CX = concentration du produit à tester inhibant 50 % de la fixation spécifique du $^3H$ dihydroalprénolol.

L'affinité relative est donnée par la relation: $ARL = 100 \frac{CD}{CX}$

On a obtenu les résultats suivants:

| Produit de l'exemple | ARL en % |
|---|---|
| 1 | 18 |
| 2 | 172 |
| 3 | 0,1 |
| 4 | 0,03 |

Ces résultats montrent que les produits de la présente demande présentent une affinité notable pour les récepteurs $\beta_2$ adrénergiques.

7) Etude de la toxicité aigüe

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants:

| Produit de l'exemple | $DL_0$ en mg/kg |
|---|---|
| 1 | > 100 |
| 2 | > 100 |
| 3 | > 100 |
| 4 | 60 |
| 5 | > 100 |
| 6 | > 400 |
| 8 | > 400 |
| 11 | 200 |

**Revendications** pour les Etats contractants BE, CH, DE, FR, GB, IT, LI, LU, NL, SE

1. Les dérivés de l'indol éthényl phénol, ainsi que leurs sels d'addition avec les acides minéraux ou organiques, caractérisés en ce qu'ils répondent à la formule générale (I):

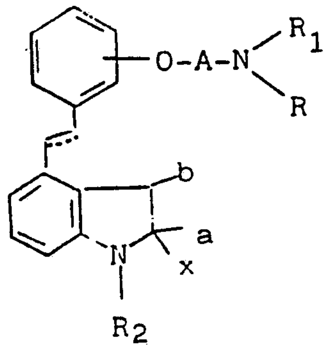

(I)

dans laquelle

R et $R_1$ représentent, indépendamment l'un de l'autre, un atomé d'hydrogène, un radical alkyle linéaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou un radical aralkyle renfermant de 7 à 12 atomes de carbone éventuellement substitué par 1, 2 ou 3 radicaux choisis parmi le groupe des halogènes, méthyle, éthyle, méthoxy, éthoxy, trifluorométhyle, méthylthio, amino et nitro, ou

R et $R_1$ forment ensemble un hétérocycle saturé choisi parmi les cycles pyrrolidino, pipéridino, morpholino, pipérazinyle, méthyl pipérazinyle, éthyl pipérazinyle ou propyl pipérazinyle,

a représente ensemble avec x une fonction oxo, ou représente ensemble avec b une liaison carbone-carbone,

x représente un atome d'hydrogène, ou ensemble avec a une fonction oxo,

b représente un atome d'hydrogène, ou ensemble avec a représente une liaison carbone-carbone,

le pointillé représente la présence éventuelle d'une liaison carbone-carbone,

A représente une chaîne

$-CH_2-CH-CH_2-$ ou $-(CH_2)_n-$
I
OH

dans laquelle

n peut prendre les valeurs 2, 3, 4 ou 5 et

$R_2$ représente un atome d'hydrogène, un radical alkyle linéaire renfermant de 1 à 5 atomes de carbone ou un radical alkyle ramifié renfermant de 3 à 5 atomes de carbone, les dérivés de formule (I) étant de configuration trans, lorsque le pointillé représente une liaison carbone-carbone, étant entendu que dans le cas où A représente une chaîne

$-CH_2-CH-CH_2-$
I
OH

R et $R_1$ ne peuvent pas représenter en même temps un atome d'hydrogène.

2. Les dérivés de l'indol éthényl phénol tels que définis par la formule (I) de la revendication 1, ainsi que leurs sels d'addition avec les acides minéraux ou organiques, caractérisés en ce que

$R_2$ représente un atome d'hydrogène,
A représente une chaîne

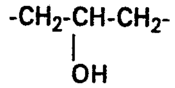

et R et $R_1$ représentent un atome d'hydrogène ou un radical alkyle, étant entendu que R et $R_1$ ne peuvent pas représenter en même temps un atome d'hydrogène.

3. Les dérivés de l'indol éthényl phénol selon la revendication 1, ainsi que leurs sels d'addition avec les acides minéraux ou organiques, caractérisés en ce que
$R_2$ représente un atome d'hydrogène,
A représente une chaîne $-(CH_2)_n-$,
n étant défini comme à la revendication 1 et
R et $R_1$ représentent un atome d'hydrogène ou un radical alkyle.

4. Les dérivés de l'indol éthényl phénol selon l'une quelconque des revendications 1 à 3, ainsi que leurs sels d'addition avec les acides minéraux ou organiques, caractérisés en ce que la chaîne latérale

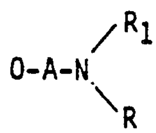

est fixée en position ortho.

5. Les dérivés de l'indol éthényl phénol selon l'une quelconque des revendications 1 à 4, ainsi que leurs sels d'addition avec les acides minéraux ou organiques, caractérisés en ce que
R représente un atome d'hydrogène et
a et b représentent ensemble une liaison carbone-carbone.

6. L'un quelconque des dérivés de formule (I), telle que définie à la revendication 1, dont les noms suivent:
- le 1-/(1,1-diméthyl éthyl)amino/-3-/4-(2-(1H-indol-4-yl)éthényl/phénoxy/2-propanol,
- le 1-/(1,1-diméthyl éthyl)amino/-3-/2/2-(1H-indol-4-yl)éthyl/phénoxy/2-propanol,
- la 1,3-dihydro 4-/2-/2-/3-/(1,1-diméthyl éthyl)amino/2-hydroxy propoxy/phényl/éthényl/2H-indol-2-one,
- la N-(1,1-diméthyl éthyl)-3-/2-2-(1H-indol-4-yl) éthyl/ phénoxy/ propanamine, ainsi que leurs sels d'addition avec les acides minéraux ou organiques.

7. Procédé de préparation des dérivés de l'indol éthényl phénol tels que définis par la formule (I) de la revendication 1, ainsi que de leurs sels, caractérisé en ce que l'on fait réagir le 4-formyl indole de formule (II):

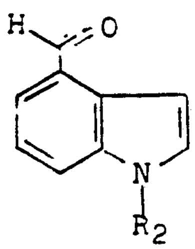

(II)

dans laquelle $R_2$ a la signification déjà indiquée, avec un halogénure d'hydroxybenzyl triphényl phosphonium de formule (III):

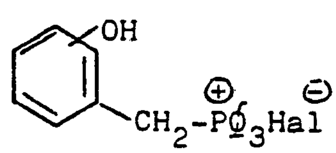

(III)

dans laquelle le radical hydroxy se trouve en position 2, 3 ou 4 et Hal représente un atome de chlore ou de brome, pour obtenir un dérivé de formule (IV):

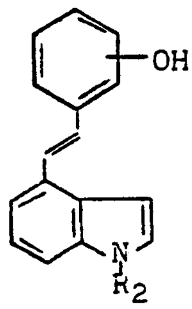

(IV)

dans laquelle $R_2$ la signification déjà indiquée, que l'on fait réagir ou bien avec l'halogénure de formule (V):

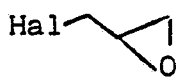

(V)

dans laquelle Hal a la signification déjà indiquée, pour obtenir un dérivé de formule (VI):

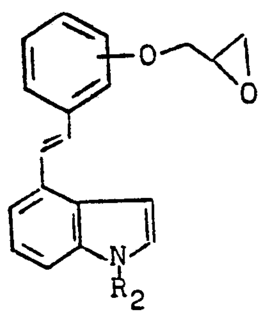

(VI)

dans laquelle $R_2$ a la signification déjà indiquée, que l'on fait réagir avec l'amine primaire ou secondaire de formule (VII):

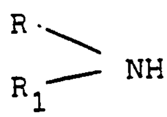

(VII)

dans laquelle R et $R_1$ ont la signification déjà indiquée, pour obtenir un produit de formule ($I_A$):

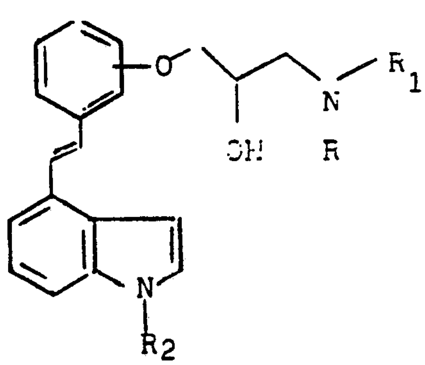

($I_A$)

dans laquelle R, $R_1$ et $R_2$ ont la signification déjà indiquée, que soit l'on isole et, si désiré, salifie, soit l'on soumet à une hydrogénation pour obtenir un produit de formule ($I_B$):

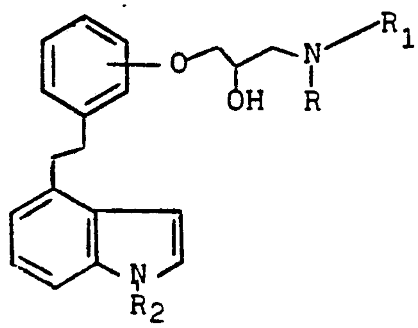

($I_B$)

dans laquelle R, $R_1$ et $R_2$ ont la signification déjà indiquée, que, si désiré, l'on salifie, <u>ou bien</u> l'on fait réagir ledit produit de formule (IV) avec l'halogénure de formule (V'):

$$Hal\text{-}(CH_2)_n\text{-}OR_3 \qquad (V')$$

dans laquelle
Hal et n ont la signification déjà indiquée et
$R_3$ représente un radical tosyle ou un atome d'hydrogène, pour obtenir un produit de formule (VI'):

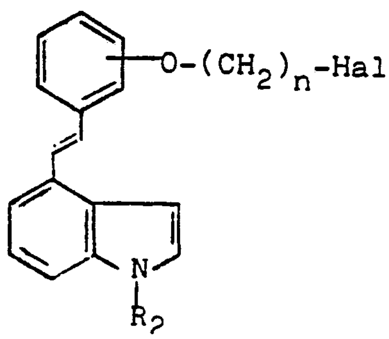

(VI')

dans laquelle n, Hal et $R_2$ ont la signification déjà indiquée, que l'on fait réagir avec l'amine de formule (VII) définie ci-dessus, pour obtenir un produit de formule ($I'_A$):

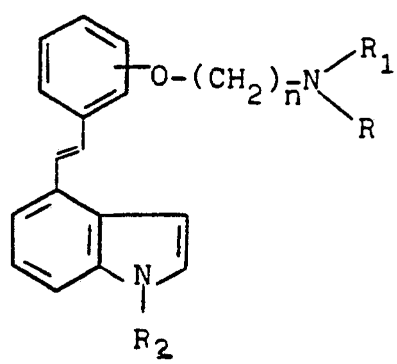

($I'_A$)

dans laquelle n, R, $R_1$ et $R_2$ ont la signification déjà indiquée, que soit l'on isole et, si désiré, salifie, soit l'on soumet à une hydrogénation pour obtenir un produit de formule ($I'_B$):

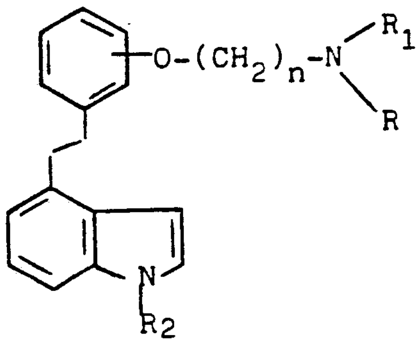

(I'$_B$)

dans laquelle n, R, $R_1$ et $R_2$ ont la signification déjà indiquée, puis soumet, si désiré, lesdits produits de formule ($I_A$), ($I_B$), ($I'_A$) et ($I'_B$) à l'action d'un agent d'halogénation pour obtenir un produit de formule (VIII):

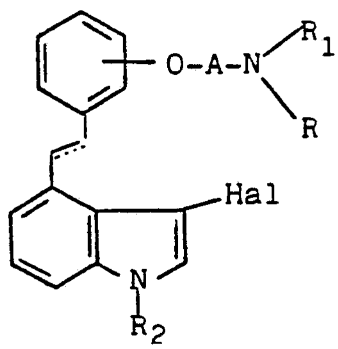

(VIII)

dans laquelle A, Hal, R, $R_1$, $R_2$ et le pointillé ont la signification déjà indiquée, que l'on soumet à une hydrolyse pour obtenir un produit de formule ($I_C$):

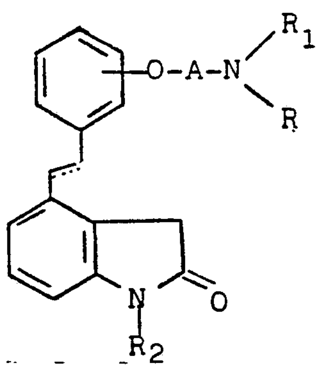

($I_C$)

dans laquelle A, R, $R_1$, $R_2$ et le pointillé ont la signification déjà indiquée, que l'on isole et, si désiré, salifie.

8. Médicaments, caractérisés en ce qu'ils sont constitués par les dérivés de l'indol éthényl phénol, tels que définis par la formule (I) de la revendication 1, ainsi que par leurs sels d'addition avec les acides pharmaceutiquement acceptables.

9. Médicaments, caractérisés en ce qu'ils sont constitués par les dérivés de l'indol éthényl phénol, tels que définis dans l'une des revendications 2 à 5, ainsi que par leurs sels d'addition avec les acides pharmaceutiquement acceptables.

10. Médicaments, caractérisés en ce qu'ils sont constitués par les dérivés de l'indol éthényl phénol, tels que définis à la revendication 6, ainsi que par leurs sels d'addition avec les acides pharmaceutiquement acceptables.

11. Compositions pharmaceutiques, caractérisées en ce qu'elles renferment, titre de principe actif, l'un au moins des médicaments, tels que définis à l'une quelconque des revendications 8, 9 ou 10.

**Revendications** pour l'Etat contractant AT

1. Procédé de préparation des dérivés de l'indol éthényl phénol et de leurs sels d'addition avec les acides minéraux ou organiques, répondant à la formule générale (I):

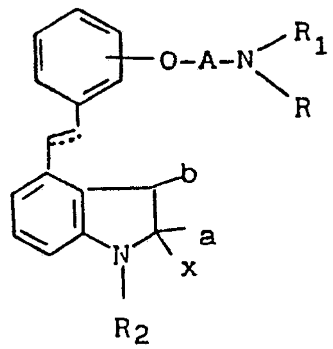

(I)

dans laquelle

R et $R_1$ représentent indépendamment l'un de l'autre, un atome d'hydrogène, un radical alkyle lineaire renfermant de 1 à 5 atomes de carbone, un radical alkyle ramifié renfermant de 3 à 5 atomes de carbone, un radical cycloalkyle renfermant de 3 à 7 atomes de carbone, un radical cycloalkylalkyle renfermant de 4 à 7 atomes de carbone, ou ur radical aralkyle renfermant de 7 à 12 atomes de carbone éventuellement substitué par 1, 2 ou 3 radicaux choisis parmi le groupe des halogènes, méthyle, éthyle, méthoxy, éthoxy, trifluorométhyle, méthylthio, amino et nitro, ou

R et $R_1$ forment ensemble un hétérocycle saturé choisi parmi les cycles pyrrolidino, pipéridino, morpholino, pipérazinyle, méthyl pipérazinyle, éthyl pipérazinyle ou propyl pipérazinyle,

a représente ensemble avec x une fonction oxo, ou représente ensemble avec b une liaison carbone-carbone,

x représente un atome d'hydrogène, ou ensemble avec a une fonction oxo,

b représente un atome d'hydrogène, ou ensemble avec a représente une liaison carbone-carbone,

le pointillé représente la présence éventuelle d'une liaison carbone-carbone,

A représente une chaîne

$-CH_2-CH-CH_2-$ ou $-(CH_2)_n-$
|
OH

dans laquelle

n peut prendre les valeurs 2, 3, 4 ou 5 et $R_2$ représente un atome d'hydrogène, un radical alkyle linéaire renfermant de 1 à 5 atomes de carbone ou un radical alkyle ramifié renfermant de 3 à 5 atomes de carbone, les dérivés de formule (I) étant de configuration trans, lorsque le pointillé représente une liaison carbone-carbone, étant entendu que dans le cas où A représente une chaîne

$-CH_2-CH-CH_2-$
|
OH

R et $R_1$ ne peuvent pas représenter en même temps un atome d'hydrogène, caractérisé en ce que l'on fait réagir le 4-formyl indole de formule (II):

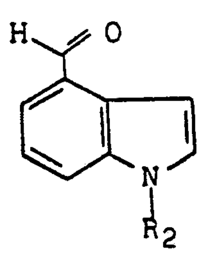

(II)

dans laquelle $R_2$ a la signification déjà indiquée, avec un halogénure d'hydroxybenzyl triphényl phosphonium de formule (III):

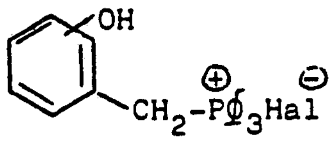

(III)

dans laquelle le radical hydroxy se trouve en position 2, 3 ou 4 et Hal représente un atome de chlore ou de brome, pour obtenir un dérivé de formule (IV):

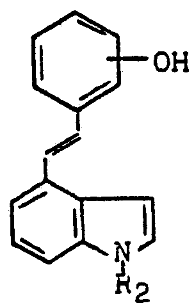

(IV)

dans laquelle $R_2$ a la signification déjà indiquée que l'on fait réagir ou bien avec l'halogénure de formule (V):

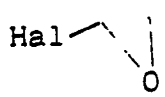

(V)

dans laquelle Hal a la signification déjà indiquée, pour obtenir un dérivé de formule (VI):

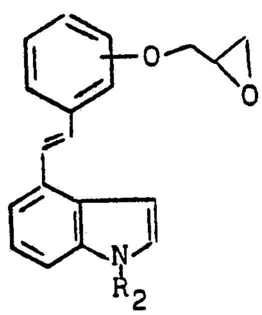

(VI)

dans laquelle $R_2$ a la signification déjà indiquée, que l'on fait réagir avec l'amine primaire ou secondaire de formule (VII):

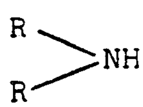

(VII)

dans laquelle R et $R_1$ ont la signification déjà indiquée, pour obtenir un produit de formule ($I_A$):

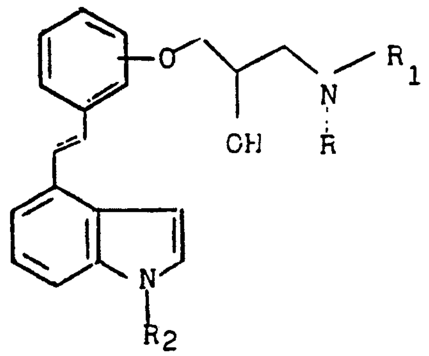

($I_A$)

dans laquelle R, $R_1$ et $R_2$ ont la signification déjà indiquée, que soit l'on isole et, si désiré, salifie, soit l'on soumet à une hydrogénation pour obtenir un produit de formule ($I_B$):

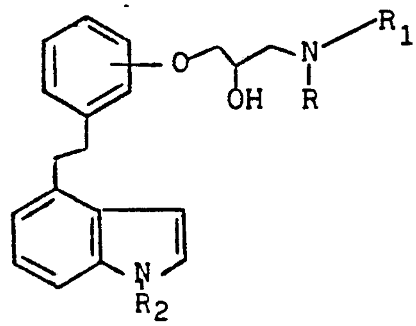

($I_B$)

dans laquelle R, $R_1$ et $R_2$ ont la signification déjà indiquée, que, si désiré, l'on salifie, ou bien l'on fait réagir ledit produit de formule (IV) avec l'halogénure de formule (V'):

$$Hal\text{-}(CH_2)_n\text{-}OR_3 \qquad (V')$$

dans laquelle
Hal et n ont la signification déjà indiquée et
$R_3$ représente un radical tosyle ou un atome d'hydrogène, pour obtenir un produit de formule (VI'):

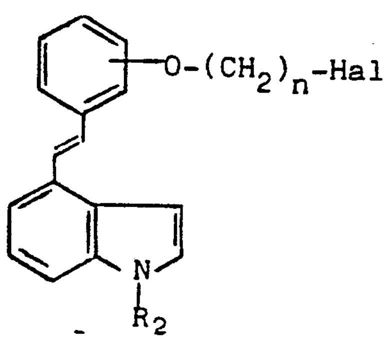

(VI')

dans laquelle n, Hal et $R_2$ ont la signification déjà indiquée, que l'on fait réagir avec l'amine de formule (VII) définie ci-dessus, pour obtenir un produit de formule ($I'_A$):

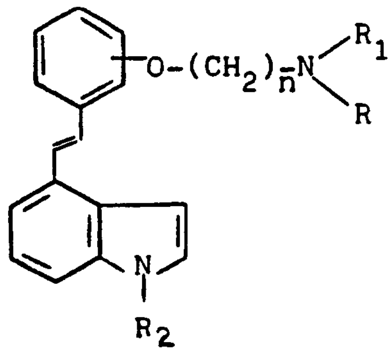

(I'$_A$)

dans laquelle n, R, $R_1$ et $R_2$ ont la signification déjà indiquée, que soit l'on isole et, si désiré, salifie, soit l'on soumet à une hydrogénation pour obtenir un produit de formule (I'$_B$):

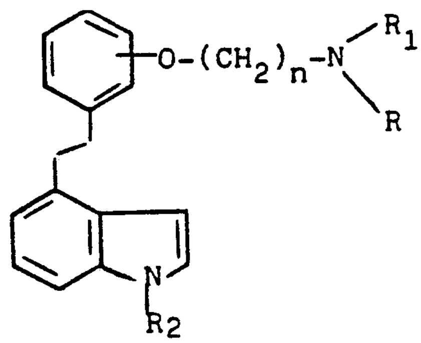

(I'$_B$)

dans laquelle n, R, $R_1$ et $R_2$ ont la signification déjà indiquée, puis soumet, si désiré, lesdits produits de formule ($I_A$), ($I_B$), (I'$_A$) et (I'$_B$) à l'action d'un agent d'halogénation pour obtenir un produit de formule (VIII):

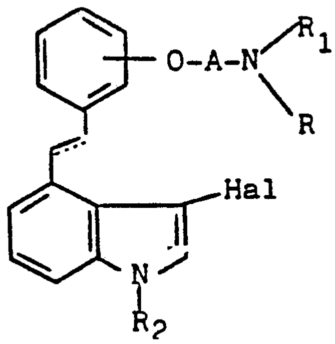

(VIII)

dans laquelle A, Hal, R, $R_1$, $R_2$ et le pointillé ont la signification déjà indiquée, que l'on soumet à une hydrolyse pour obtenir un produit de formule ($I_C$):

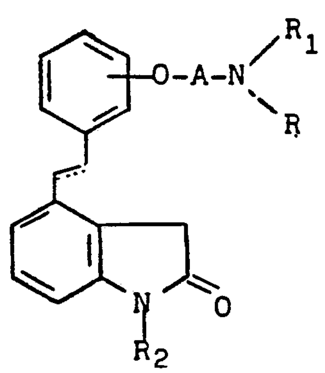

($I_C$)

dans laquelle A, R, $R_1$, $R_2$ et le pointillé ont la signification déjà indiquée, que l'on isole et, si désiré, salifie.

2. Procédé selon la revendication 1, caractérisé en ce que l'on utilise au départ un dérivé de formule (II) dans laquelle $R_2$ représente un atome d'hydrogène, un halogénure de formule (V) puis une amine de formule (VII)

dans laquelle R et $R_1$ représentent un atome d'hydrogène ou un radical alkyle, étant entendu que R et $R_1$ ne peuvent pas représenter en même temps un atome d'hydrogène.

3. Procédé selon la revendication 1, caractérisé en ce que l'on utilise au départ un dérivé de formule (II) dans laquelle $R_2$ représente un atome d'hydrogène, un halogénure de formule (V') puis une amine de formule (VII) dans laquelle R et $R_1$ représentent un atome d'hydrogène ou un radical alkyle.

4. Procédé selon l'une quelconque des revendications 1 à 3, caractérisé en ce que l'on utilise au départ un dérivé de formule (III) dans laquelle le substituant OH est en position ortho.

5. Procédé selon l'une quelconque des revendications 1 à 4, caractérisé en ce que l'on prépare des dérivés de formule (I) dans laquelle

R représente un atome d'hydrogène et

a et b représentent ensemble une liaison carbone-carbone.

6. Procédé selon la revendication 1, caractérisé en ce que l'on prépare l'un quelconque des dérivés de formule (I), telle que définie à la revendication 1, dont les noms suivent:

- le 1-/(1,1-diméthyl éthyl)amino/-3-/4-(2-(1H-indol-4-yl)éthényl/phénoxy/2-propanol,
- le 1-/(1,1-diméthyl éthyl)amino/-3-/2/2-(1H-indol-4-yl)éthyl/phénoxy/2-propanol,
- la 1,3-dihydro 4-/2-/2-/3-/(1,1-diméthyl éthyl)amino/2-hydroxy propoxy/phényl/éthényl/2H-indol-2-one,
- la N-(1,1-diméthyl éthyl)-3-/2-2-(1H-indol-4-yl) éthyl/ phénoxy/ propanamine, ainsi que leurs sels d'addition avec les acides minéraux ou organiques.

**Patentansprüche** für die Vertragsstaaten: BE, CH, DE, FR, GB, IT, LI, LU, NL, SE

1. Indolethenylphenol-Derivate sowie deren Additionssalze mit Mineral- oder organischen Säuren, dadurch gekennzeichnet, daß sie der allgemeinen Formel (I) entsprechen:

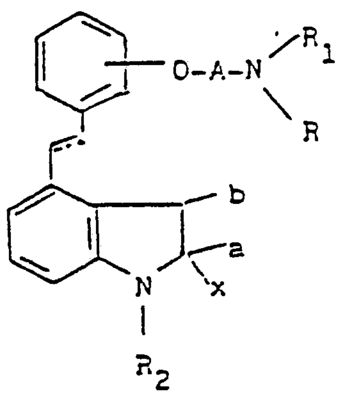

(I)

worin

R und $R_1$ unabhängig voneinander ein Wasserstoffatom, einen linearen Alkylrest mit 1 bis 5 Kohlenstoffatomen, einen verzweigten Alkylrest mit 3 bis 5 Kohlenstoffatomen, einen Cycloalkylrest mit 3 bis 7 Kohlenstoffatomen, einen Cycloalkalkylrest mit 4 bis 7 Kohlenstoffatomen oder einen Aralkylrest mit 7 bis 12 Kohlenstoffatomen, gegebenenfalls substituiert durch 1, 2 oder 3 Reste, ausgewählt unter der Gruppe der Halogene, Methyl, Ethyl, Methoxy, Ethoxy, Trifluormethyl, Methylthio, Amino und Nitro, bedeuten oder

R und $R_1$ gemeinsam einen gesättigten Heterocyclus, ausgewählt unter den Pyrrolidino-, Piperidino-, Morpholino-, Piperazinyl-, Methylpiperazinyl-, Ethylpiperazinyl- oder Propylpiperazinylringen, bilden;

a gemeinsam mit x eine Oxofunktion darstellt oder gemeinsam mit b eine Kohlenstoff-Kohlenstoff-Bindung wiedergibt;

x ein Wasserstoffatom darstellt oder gemeinsam mit a eine Oxofunktion wiedergibt;

b ein Wasserstoffatom darstellt oder gemeinsam mit a eine Kohlenstoff-Kohlenstoff-Bindung bildet;

die gestrichelte Linie die etwaige Anwesenheit einer Kohlenstoff-Kohlenstoff-Bindung anzeigt;

A eine Kette

$$-CH_2-\underset{\displaystyle OH}{\underset{|}{CH}}-CH_2- \text{ oder } -(CH_2)_n-$$

wiedergibt, worin

n die Werte 2, 3, 4 oder 5 annehmen kann; und

$R_2$ ein Wasserstoffatom, einen linearen Alkylrest mit 1 bis 5 Kohlenstoffatomen oder einen verzweigten Alkylrest mit 3 bis 5 Kohlenstoffatomen bedeutet, wobei die Derivate der Formel (I) die trans-Konfiguration besitzen, wenn die gestrichelte Linie eine Kohlenstoff-Kohlenstoff-Bindung wiedergibt, mit der Maßgabe, daß,

wenn A eine Kette

$CH_2$-CH-$CH_2$-
|
OH

bedeutet, R und $R_1$ nicht gleichzeitig ein Wasserstoffaton darstellen können.

2. Indolethenylphenol-Derivate der Formel (I) gemäß Anspruch 1 sowie deren Additionssalze mit Mineral- oder organischen Säuren, dadurch gekennzeichnet, daß

$R_2$ ein Wasserstoffatom bedeutet,

A für eine Kette

-$CH_2$-CH-$CH_2$-
|
OH

steht und

R und $R_1$ ein Wasserstoffatom oder einen Alkylrest bedeuten, mit der Maßgabe, daß R und $R_1$ nicht gleichzeitig ein Wasserstoffatom darstellen können.

3. Indolethenylphenol-Derivate gemäß Anspruch 1 sowie deren Additionssalze mit Mineral- oder organischen Säuren, dadurch gekennzeichnet, daß

$R_2$ ein Wasserstoffatom bedeutet,

A für eine Kette $-(CH_2)_n-$ steht, wobei n wie in Anspruch 1 definiert ist, und

R und $R_1$ ein Wasserstoffatom oder einen Alkylrest bedeuten.

4. Indolethenylphenol-Derivat gemäß einem der Ansprüche 1 bis 3 sowie deren Additionssalze mit Mineral- oder organischen Säuren, dadurch gekennzeichnet, daß die Seitenkette

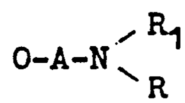

in ortho-Stellung gebunden ist.

5. Indolethenylphenol-Derivate gemäß einem der Ansprüche 1 bis 4 sowie deren Additionssalze mit Mineral- oder organischen Säuren, dadurch gekennzeichnet, daß

R ein Wasserstoffatom bedeutet und

a und b gemeinsam eine Kohlenstoff-Kohlenstoff-Bindung wiedergeben.

6. Eines der Derivate der Formel (I) gemäß Anspruch 1 mit den folgenden Bezeichnungen

1-[(1,1-Dimethylethyl)-amino]-3-[4-[2-(1H-indol-4-yl)-ethenyl]-phenoxy]-2-propanol,

1-[(1,1-Dimethylethyl)-amino]-3-[2-[2-(1H-indol-4-yl)-ethyl]-phenoxy]-2-propanol,

1,3-Dihydro-4-[2-[2-[3-[(1,1-dimethylethyl)-amino]-2-hydroxypropoxy]-phenyl]-ethenyl]-2H-indol-2-on,

N-(1,1-Dimethylethyl)-3-[2-[2-(1H-indol-4-yl)-ethyl]-phenoxy]-propanamin

sowie deren Additionssalze mit Mineral- oder organischen Säuren.

7. Verfahren zur Herstellung von Indolethenylphenol-Derivaten der Formel (I) gemäß Anspruch 1 sowie von deren Salzen, dadurch gekennzeichnet, daß man 4-Formylindol der Formel (II)

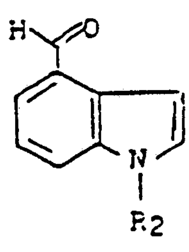

(II)

worin $R_2$ die angegebene Bedeutung besitzt, mit einem Hydroxybenzyltriphenylphosphoniumhalogenid der Formel (III)

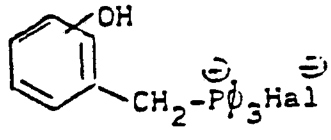

(III)

worin sich der Hydroxyrest in 2-, 3- oder 4-Stellung befindet und Hal ein Chlor- oder Bromatom darstellt, umsetzt, um ein Derivat der Formel (IV)

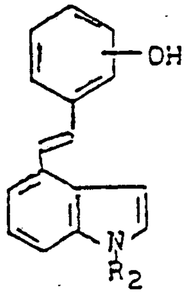

(IV)

zu erhalten, worin $R_2$ die angegebene Bedeutung besitzt, das man umsetzt entweder mit den Halogenid der Formel (V)

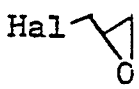

(V)

worin Hal die angegebene Bedeutung besitzt, um ein Derivat der Formel (VI)

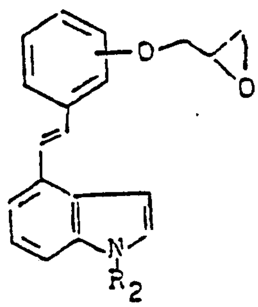

(VI)

zu erhalten, worin $R_2$ die angegebene Bedeutung besitzt, das man mit dem primären oder sekundären Amin der Formel (VII)

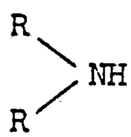

(VII)

worin R und $R_1$ die angegebene Bedeutung besitzen, umsetzt, um ein Produkt der Formel ($I_A$)

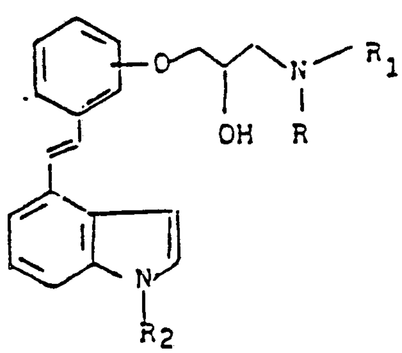

($I_A$)

zu erhalten, worin R, $R_1$ und $R_2$ die angegebene Bedeutung besitzen, das man entweder isoliert und gewünschtenfalls in ein Salz überführt oder einer Hydrierung unterzieht, um ein Produkt der Formel ($I_B$)

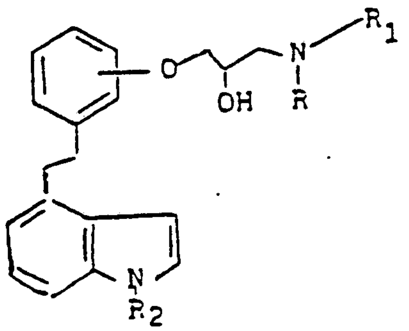

($I_B$)

zu erhalten, worin R, $R_1$ und $R_2$ die angegebene Bedeutung besitzen, das man gewünschtenfalls in ein Salz überführt,
<u>oder</u> das Produkt der Formel (IV) mit dem Halogenid der Formel (V')

$$Hal\text{-}(CH_2)_n\text{-}OR_3 \qquad (V')$$

umsetzt, worin
Hal und n die angegebene Bedeutung besitzen und
$R_3$ einen Tosylrest oder ein Wasserstoffatom bedeutet, um ein Produkt der Formel (VI')

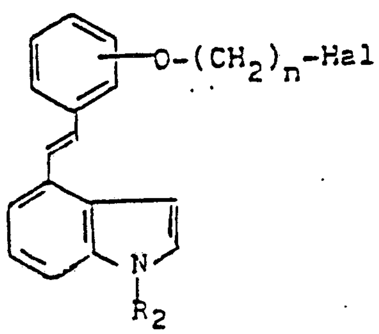

(VI')

zu erhalten, worin n, Hal und $R_2$ die angegebene Bedeutung besitzen, welches man mit dem Amin der vorstehenden Formel (VII) umsetzt, um ein Produkt der Formel ($I'_A$)

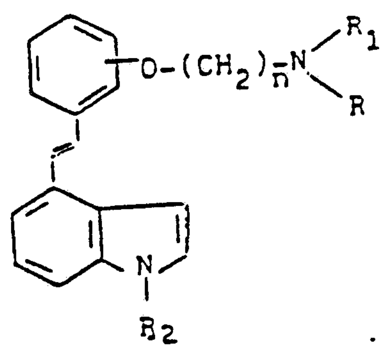

($I'_A$)

zu erhalten, worin n, R, $R_1$ und $R_2$ die angegebene Bedeutung besitzen, das man isoliert und gewünschtenfalls in ein Salz überführt, oder einer Hydrierung unterzieht, um ein Produkt der Formel ($I'_B$)

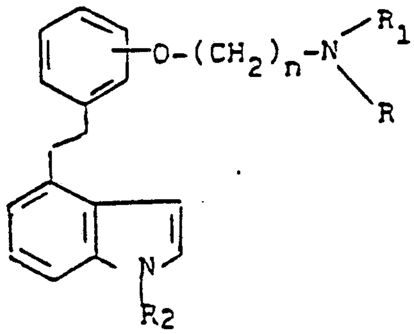

($I'_B$)

zu erhalten, worin n, R, $R_1$ und $R_2$ die angegebene Bedeutung besitzen, danach gewünschtenfalls die Produkte der Formeln ($I_A$), ($I_B$), ($I'_A$) und ($I'_B$) der Einwirkung eines Halogenierungsmittels unterzieht, um ein Produkt der Formel (VIII)

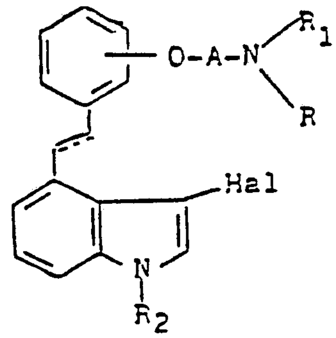

(VIII)

zu erhalten, worin A, Hal, R, $R_1$, $R_2$ und die gestrichelte Linie die angegebene Bedeutung besitzen, welches man einer Hydrolyse unterzieht, um ein Produkt der Formel ($I_C$)

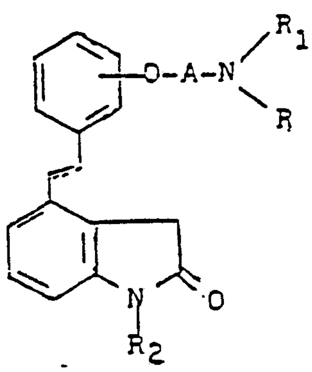

($I_C$)

zu erhalten, worin A, R, $R_1$, $R_2$ und die gestrichelte Linie die angegebene Bedeutung besitzen, das man isoliert und gewünschtenfalls in ein Salz überführt.

8. Arzneimittel, dadurch gekennzeichnet, daß sie aus den Indolethenylphenol-Derivaten der Formel (I) gemäß Anspruch 1 sowie aus deren Additionssalzen mit pharmazeutisch verträglichen Säuren bestehen.

9. Arzneimittel, dadurch gekennzeichnet, daß sie aus den Indolethenylphenol-Derivaten gemäß einem der Ansprüche 2 bis 5 sowie aus deren Additionssalzen mit pharmazeutisch verträglichen Säuren bestehen.

10. Arzneimittel, dadurch gekennzeichnet, daß sie aus den Indolethenylphenol-Derivaten gemäß Anspruch 6 sowie aus deren Additionssalzen mit pharmazeutisch verträglichen Säuren bestehen.

11. Pharmazeutische Zusammensetzungen, dadurch gekennzeichnet, daß sie als Wirkstoff zumindest eines der Arzneimittel gemäß einem der Ansprüche 8, 9 oder 10 enthalten.

**Patentansprüche** für den Vertragsstaat: AT

1. Verfahren zur Herstellung von Indolethenylphenol-Derivaten und deren Additionssalzen mit Mineral- oder organischen Säuren der allgemeinen Formel (I)

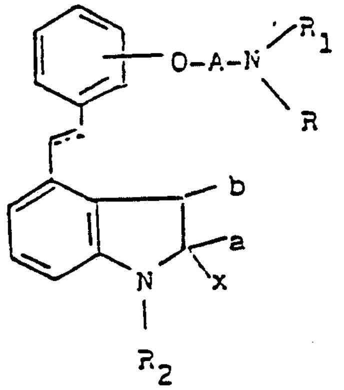

(I)

worin

R und $R_1$ unabhängig voneinander ein Wasserstoffatom, einen linearen Alkylrest mit 1 bis 5 Kohlenstoffatomen, einen verzweigten Alkylrest mit 3 bis 5 Kohlenstoffatomen, einen Cycloalkylrest mit 3 bis 7 Kohlenstoffatomen, einen Cycloalkalkylrest mit 4 bis 7 Kohlenstoffatomen oder einen Aralkylrest mit 7 bis 12 Kohlenstoffatomen, gegebenenfalls substituiert durch 1, 2 oder 3 Reste, ausgewählt unter der Gruppe der Halogene, Methyl, Ethyl, Methoxy, Ethoxy, Trifluormethyl, Methylthio, Amino und Nitro, bedeuten oder

R und R gemeinsam einen gesättigten Heterocyclus, ausgewählt unter den Pyrrolidino-, Piperidino-, Morpholino-, Piperazinyl-, Methylpiperazinyl-, Ethylpiperazinyl- oder Propylpiperazinylringen, bilden;

a gemeinsam mit x eine Oxofunktion darstellt oder gemeinsam mit b eine Kohlenstoff-Kohlenstoff-Bindung wiedergibt;

x ein Wasserstoffatom darstellt oder gemeinsam mit a eine Oxofunktion wiedergibt;

b ein Wasserstoffatom darstellt oder gemeinsam mit a eine Kohlenstoff-Kohlenstoff-Bindung bildet;

die gestrichelte Linie die etwaige Anwesenheit einer Kohlenstoff-Kohlenstoff-Bindung anzeigt,

A eine Kette

$$-CH_2-\underset{\displaystyle OH}{\underset{|}{CH}}-CH_2- \text{ oder } -(CH_2)_n-$$

wiedergibt, worin

n die Werte 2, 3, 4 oder 5 annehmen kann; und

$R_2$ ein Wasserstoffatom, einen linearen Alkylrest mit 1 bis 5 Kohlenstoffatomen oder einen verzweigten Alkylrest mit 3 bis 5 Kohlenstoffatomen bedeutet, wobei die Derivate der Formel (I) die trans-Konfiguration besitzen, wenn die gestrichelte Linie eine Kohlenstoff-Kohlenstoff-Bindung wiedergibt, mit der Maßgabe, daß, wenn A eine Kette

$$CH_2-\underset{\displaystyle OH}{\underset{|}{CH}}-CH_2-$$

bedeutet, R und $R_1$ nicht gleichzeitig ein Wasserstoffatom darstellen können, dadurch gekennzeichnet, daß man 4-Formylindol der Formel (II)

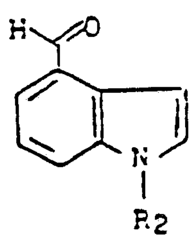

(II)

worin $R_2$ die angegebene Bedeutung besitzt, mit einem Hydroxybenzyltriphenylphosphoniumhalogenid der Formel (III)

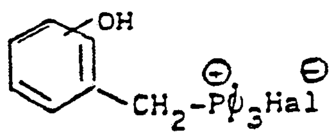

(III)

worin sich der Hydroxyrest in 2-, 3- oder 4-Stellung befindet und Hal ein Chlor- oder Bromatom darstellt, umsetzt, um ein Derivat der Formel (IV)

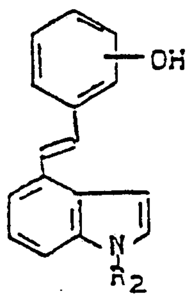

(IV)

zu erhalten, worin $R_2$ die angegebene Bedeutung besitzt, das man umsetzt
entweder mit den Halogenid der Formel (V)

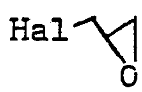

(V)

worin Hal die angegebene Bedeutung besitzt, um ein Derivat der Formel (VI)

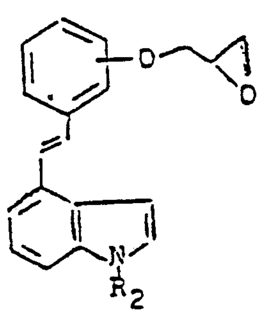

(VI)

zu erhalten, worin $R_2$ die angegebene Bedeutung besitzt, das man mit dem primären oder sekundären Amin der Formel (VII)

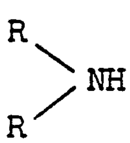

(VII)

worin R und $R_1$ die angegebene Bedeutung besitzen, umsetzt, um ein Produkt der Formel ($I_A$)

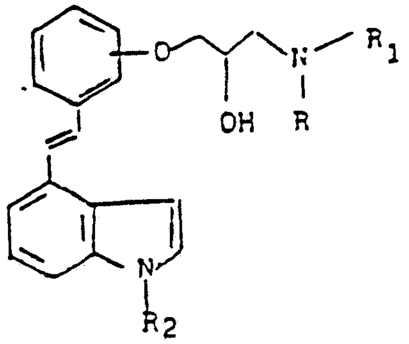

$(I_A)$

zu erhalten, worin R, $R_1$ und $R_2$ die angegebene Bedeutung besitzen, das man entweder isoliert und gewünschtenfalls in ein Salz überführt oder einer Hydrierung unterzieht, um ein Produkt der Formel $(I_B)$

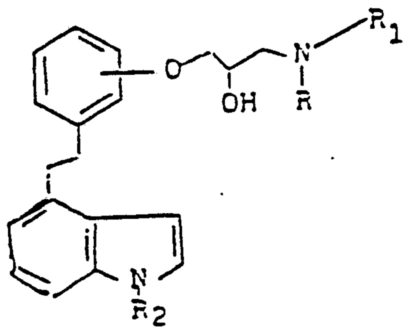

$(I_B)$

zu erhalten, worin R, $R_1$ und $R_2$ die angegebene Bedeutung besitzen, das man gewünschtenfalls in ein Salz überführt,
oder das Produkt der Formel (IV) mit dem Halogenid der Formel (V')

$$Hal\text{-}(CH_2)_n\text{-}OR_3 \qquad (V')$$

umsetzt, worin
Hal und n die angegebene Bedeutung besitzen und
$R_3$ einen Tosylrest oder ein Wasserstoffatom bedeutet, um ein Produkt der Formel (VI')

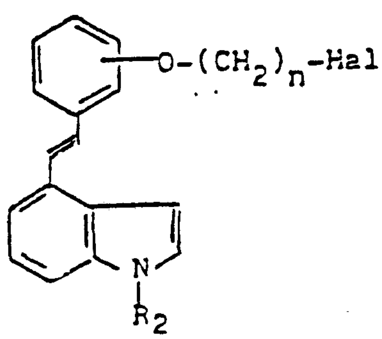

(VI')

zu erhalten, worin n, Hal und $R_2$ die angegebene Bedeutung besitzen, welches man mit dem Amin der vorstehenden Formel (VII) umsetzt, um ein Produkt der Formel $(I'_A)$

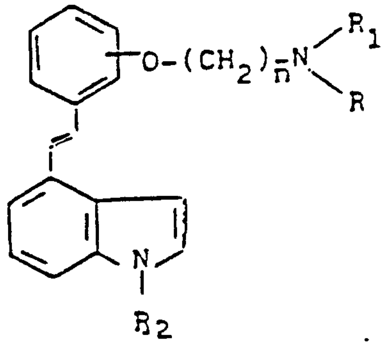

$(I'_A)$

zu erhalten, worin n, R, $R_1$ und $R_2$ die angegebene Bedeutung besitzen, das man isoliert und gewünschtenfalls in ein Salz überführt, oder einer Hydrierung unterzieht, um ein Produkt der Formel $(I'_B)$

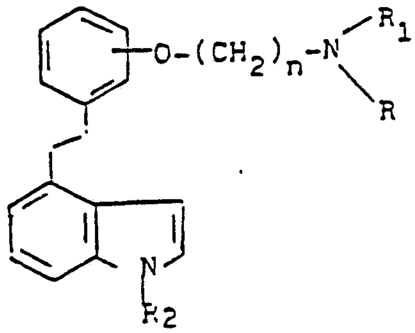

$(I'_B)$

zu erhalten, worin n, R, $R_1$ und $R_2$ die angegebene Bedeutung besitzen, danach gewünschtenfalls die Produkte der Formeln $(I_A)$, $(I_B)$, $(I'_A)$ und $(I'_B)$ der Einwirkung eines Halogenierungsmittels unterzieht, um ein Produkt der Formel (VIII)

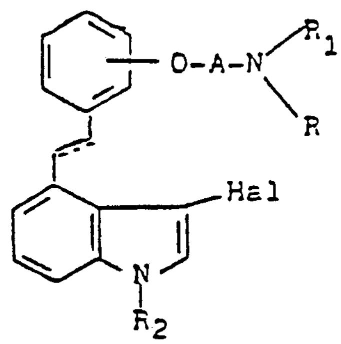

(VIII)

zu erhalten, worin A, Hal, R, $R_1$, $R_2$ und die gestrichelte Linie die angegebene Bedeutung besitzen, welches man einer Hydrolyse unterzieht, um ein Produkt der Formel $(I_C)$

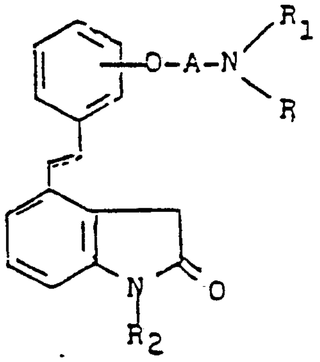

$(I_C)$

zu erhalten, worin A, R, $R_1$, $R_2$ und die gestrichelte Linie die angegebene Bedeutung besitzen, das man isoliert und gewünschtenfalls in ein Salz überführt.

2. Verfahren gemäß Anspruch 1, dadurch gekennzeichnet, daß man von einem Derivat der Formel (II), worin $R_2$ für ein Wasserstoffatom steht, einem Halogenid der Formel (V) und dann einem Amin der Formel (VII), worin R und $R_1$ ein Wasserstoffatom oder einen Alkylrest darstellen, ausgeht, mit der Maßgabe, daß R und $R_1$ nicht gleichzeitig ein Wasserstoffatom darstellen können.

3. Verfahren gemäß Anspruch 1, dadurch gekennzeichnet, daß man von einem Derivat der Formel (II), worin $R_2$ für ein Wasserstoffatom steht, einem Halogenid der Formel (V') und dann einem Amin der Formel (VII), worin R und $R_1$ für ein Wasserstoffatom oder einen Alkylrest stehen, ausgeht.

4. Verfahren gemäß einem der Ansprüche 1 bis 3, dadurch gekennzeichnet, daß man von einem Derivat der Formel (III) ausgeht, worin der Substituent OH sich in ortho-Stellung befindet.

5. Verfahren gemäß einem der Ansprüche 1 bis 4, dadurch gekennzeichnet, daß man Derivate der Formel (I) herstellt, worin
R für ein Wasserstoffatom steht und
a und b gemeinsam eine Kohlenstoff-Kohlenstoff-Bindung wiedergeben.

6. Verfahren gemäß Anspruch 1, dadurch gekennzeichnet, daß man eines der Derivate der Formel (I) gemäß Anspruch 1 mit den folgenden Bezeichnungen
1-[(1,1-Dimethylethyl)-amino]-3-[4-[2-(1H-indol-4-yl)-ethenyl]-phenoxy]-2-propanol,
1-[(1,1-Dimethylethyl)-amino]-3-[2-[2-(1H-indol-4-yl)-ethyl]-phenoxy]-2-propanol,
1,3-Dihydro-4-[2-[2-[3-[(1,1-dimethylethyl)-amino]-2-hydroxypropoxy]-phenyl]-ethenyl]-2H-indol-2-on,
N-(1,1-Dimethylethyl)-3-[2-[2-(1H-indol-4-yl)-ethyl]-phenoxy]-propanamin
sowie deren Additionssalze mit Mineral- oder organischen Säuren herstellt.

**Claims** for the Contracting States: BE, CH, DE, FR, GB, IT, LI, LU, NL, SE

1. Derivatives of ethenylphenol indole, as well as their addition salts with mineral to organic acids, characterized in that they correspond to the general formula (I):

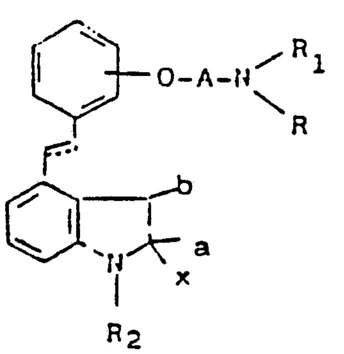

(I)

in which
R and $R_1$ represent, independently of each other, a hydrogen atom, a linear alkyl radical containing from 1 to 5 carbon atoms, a branched alkyl radical containing from 3 to 5 carbon atoms, a cycloalkyl radical containing from 3 to 7 carbon atoms, a cycloalkylalkyl radical containing from 4 to 7 carbon atoms, or an aralkyl radical containing from 7 to 12 carbon atoms, possibly substituted by 1, 2 or 3 radicals chosen from the following group of halogens:- methyl, ethyl, methoxy, ethoxy, trifluoromethyl, methylthio, amino and nitro, or
R and $R_1$ together form a saturated heterocycle chosen from the following cycles:- pyrrolidino, piperidino, morpholino, piperazinyl, methyl piperazinyl, ethyl piperazinyl or propyl piperazinyl,
a represents, together with x, an oxo function, or represents, together with b, a carbon-carbon bond,
x represents a hydrogen atom, or together with a an oxo function,
b represents a hydrogen atom, or together with a represents a carbon-carbon bond,
the broken line represents the possible presence of a carbon-carbon bond,
A represents a

$-CH_2-\underset{\displaystyle OH}{\underset{|}{CH}}-CH_2-$ or $-(CH_2)_n-$

chain in which
n can take the values 2, 3, 4 or 5 and
$R_2$ represents a hydrogen atom, a linear alkyl radical containing from 1 to 5 carbon atoms or a branched alkyl radical containing from 3 to 5 carbon atoms, the derivatives with the formula (I) being of trans configuration when the broken line represents a carbon-carbon bond, it being understood that when A represents a

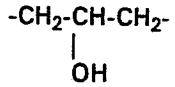

chain, R and $R_1$ cannot represent at the same time a hydrogen atom.

2. Derivatives of ethenylphenol indole as defined by formula (I) of claim 1, as well as their addition salts with mineral or orgamic acids, characterized in that

$R_2$ represents a hydrogen atom,

A represents a

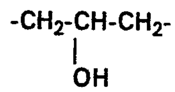

chain and

R and $R_1$ each represents a hydrogen atom or an alkyl radical, it being understood that R and $R_1$ cannot represent at the same time a hydrogen atom.

3. Derivatives of ethenylphenol indole according to claim 1, as well as their addition salts with mineral or organic acids, characterized in that

$R_2$ represents a hydrogen atom,

A represents a $-(CH_2)_n-$ chain,

n being defined as in claim 1, -and

R and $R_1$ each represent a hydrogen atom or an alkyl radical.

4. Derivatives of ethenylphenol indole according to any one of the claims 1 to 3, as well as their addition salts with mineral or organic acids, characterized in that the lateral chain

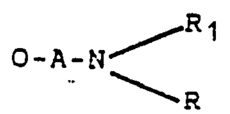

is fixed in the ortho position.

5. Derivatives of ethenylphenol indole according to any one of the claims 1 to 4, as well as their salts of addition with mineral or organic acids, characterized in that

R represents a hydrogen atom and

a and b represent together a carbon-carbon bond.

6. Any of the derivatives with the formula (I) as defined in claim 1, the names of which follow:

- 1-[(1,1-dimethylethyl)amino]-3-[4-[2-(1H-indol-4-yl)ethenyl]phenoxy]-2-propanol,
- 1-[(1,1-dimethylethyl)amino]-3-[2-[2-(1H-indol-4-yl)ethyl]phenoxy]-2-propanol,
- 1,3-dihydro-4-[2-[2-[3-[(1,1-dimethylethyl)amino]-2-hydroxypropoxy]phenyl]ethenyl]-2H-indol-2-one,
- N-(1,1-dimethylethyl)-3-[2-[2-(1H-indol-4-yl)ethyl)-phenoxy]propanamine,

as well as their addition salts with mineral or organic acids.

7. Preparation process for the derivatives of ethenylphenol indole as defined by formula (I) of claim 1, as well as for their salts, characterized in that the 4-formyl indole of formula (II):

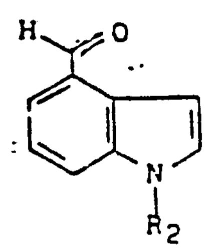

(II)

in which $R_2$ has the already indicated significance, is made to react with a hydroxybenzyltriphenyl phosphonium halogenide with the formula (III):

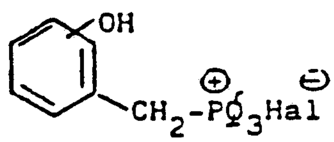

(III)

in which the hydroxy radical is found in position 2, 3 or 4 and Hal represents a chlorine or bromine atom, in order to obtain a derivative with the formula (IV):

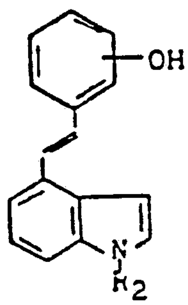

(IV)

in which

$R_2$, has the significance already indicated, which is made to react

<u>either</u> with the halogenide with the formula (V):

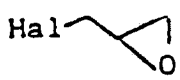

(V)

in which Hal has the significance already indicated, in order to obtain a derivative with the formula (VI):

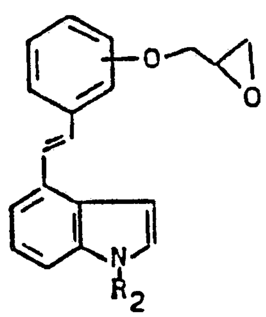

(VI)

in which $R_2$ has the significance already given, which is made to react with the primary or secondary amine with the formula (VII):

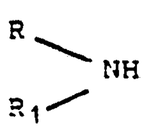

(VII)

in which R and $R_1$ have the significance already indicated, in order to obtain a product with the formula ($I_A$):

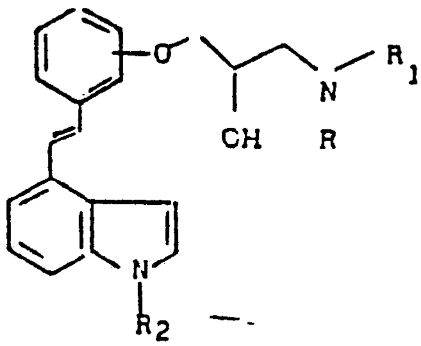

$(I_A)$

in which R, $R_1$ and $R_2$, have the significance already indicated, which either is isolated and, if desired, salified, or is submitted to a hydrogenation in order to obtain a product with the formula $(I_B)$:

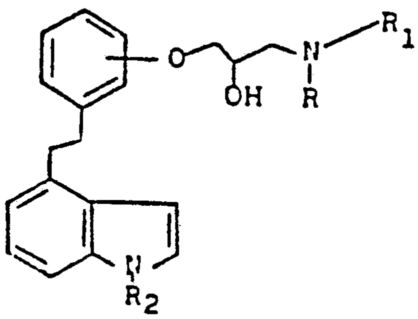

$(I_B)$

in which R, $R_1$ and $R_2$ have the significance already indicated, which, if desired, is salified,
<u>or</u> the said product with the formula (IV) is made to react with the halogenide with the formula (V'):

$$Hal\text{-}(CH_2)_n\text{-}OR_3 \qquad (V')$$

in which
Hal and n have the significance already given and
$R_3$ represents a tosyl radical or a hydrogen atom, in order to obtain a product with the formula (VI'):

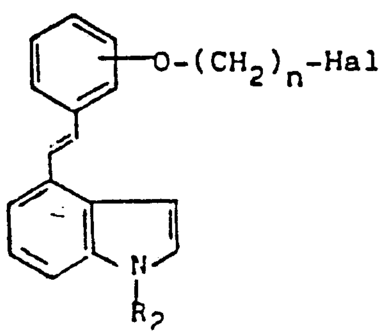

(VI')

in which n, Hal and $R_2$ have the already indicated significance, which is made to react with the amine with the formula (VII) defined above, in order to obtain a product with the formula $(I'_A)$:

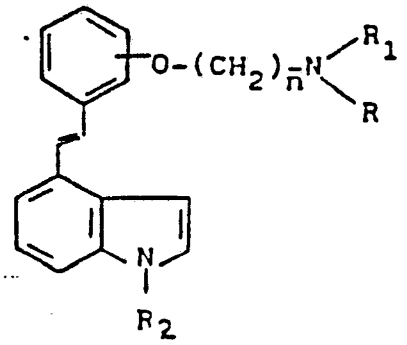

(I'A)

in which n, R, $R_1$ and $R_2$ have the significance already indicated, which either is isolated and, if desired, salified, or is submitted to a hydrogenation in order to obtain a product with the formula (I'B):

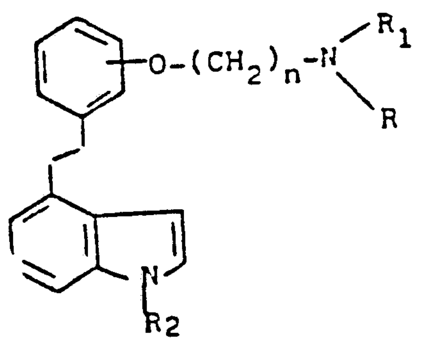

(I'B)

in which n, R, $R_1$ and $R_2$ have the significance already indicated, then, if desired, the said products with the formule ($I_A$), ($I_B$), ($I'_A$) and ($I'_B$) are submitted to the action of a halogenizing agent in order to obtain a product with the formula (VIII):

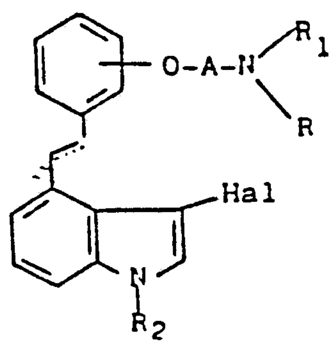

(VIII)

in which A, Hal, R, $R_1$, $R_2$ and the broken line have the significance already indicated, which is submitted to a hydrolysis in order to obtain a product with the formula ($I_C$):

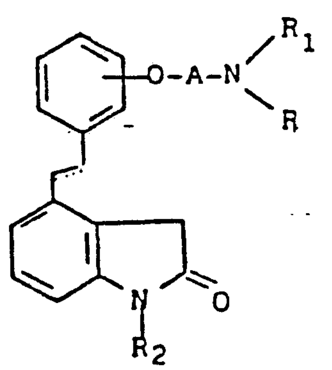

($I_C$)

in which A, R, $R_1$, $R_2$ and the broken line have the significance already indicated, which is isolated and, if desired, salified.

8. Medicaments, characterized in that they are consitituted by the derivatives of ethenylphenol indole, as defined by the formula (I) of claim 1, as well as by their addition salts with pharmaceutically acceptable acids.

9. Medicaments, characterized in that they are constituted by the derivatives of ethenylphenol indole, as defined in one of claims 2 to 5, as well as by their addition salts with pharmaceutically acceptable acids.

10. Medicaments, characterized in that they are constituted by the derivatives of ethenylphenol indole, as defined in claim 6, as well as by their addition salts with pharmaceutically acceptable acids.

11. Pharmaceutical compositions, characterized in that they contain, as active principle, one at least of the medicaments as defined in any one of the claims 8, 9 or 10.

**Claims** for the Contracting State AT

1. Preparation process for derivatives of ethenylphenol indole and of their addition salts with mineral or organic acids, corresponding to the general formula (I):

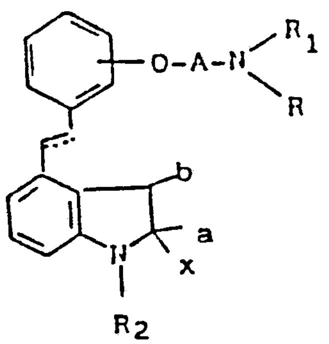

(I)

in which

R and $R_1$ represent, independently of each other, a hydrogen atom, a linear alkyl radical containing from 1 to 5 carbon atoms, a branched alkyl radical containing from 3 to 5 carbon atoms a cycloalkyl radical containing from 3 to 7 carbon atoms, a cycloalkylalkyl radical containing from 4 to 7 carbon atoms, or an aralkyl radical containing from 7 to 12 carbon atoms possibly substituted by 1, 2 or 3 radicals chosen from the following group of halogens:- methyl, ethyl, methoxy, ethoxy, trifluoromethyl, methylthio, amino and nitro, or

R and $R_1$ together form a saturated heterocycle chosen from the following cycles:- pyrrolidino, piperidino, morpholino, piperazinyl, methyl piperazinyl, ethyl piperazinyl or propyl piperazinyl,

a represents, together with x, an oxo functicn, or represents, together with b, a carbon-carbon bond,

x represents a hydrogen atom, or together with a an oxo function,

b represents a hydrogen atom, or together with a represents a carbon-carbon bond,

the broken line represents the possible presence of a carbon-carbon bond,

A represents a

$-CH_2-CH(OH)-CH_2-$ or $-(CH_2)_n-$

chain in which

n can take the values 2, 3, 4 or 5 and

$R_2$ represents a hydrogen atom, a linear alkyl radical containing from 1 to 5 carbon atoms or a branched alkyl radical containing from 3 to 5 carbon atoms, the derivatives with the formula (I) being of trans configuration when the broken line represents a carbon-carbon bond, it being understood that when A represents a

$-CH_2-CH(OH)-CH_2-$

chain, R and $R_1$ cannot represent at the same time a hydrogen atom, characterized in that the 4-formyl indole with the formula (II):

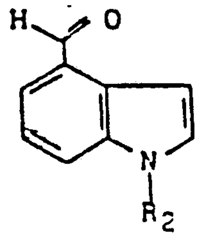

(II)

in which $R_2$ has the significance already indicated, is made to react with a hydroxybenzyl triphenyl phosphonium halogenide with the formula (III):

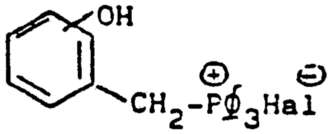

(III)

in which the hydroxy radical is found in position 2, 3 or 4 and Hal represents a chlorine or bromine atom, in order to obtain a derivative with the formula (IV):

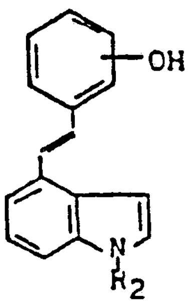

(IV)

in which $R_2$ has the significance already indicated, which is made to react

<u>either</u> with a halogenide with the formula (V):

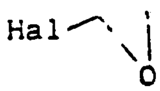

(V)

in which Hal has the significance already indicated, in order to obtain a derivative with the formula (VI):

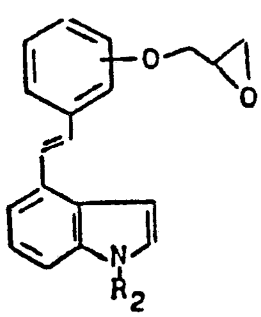

(VI)

in which $R_2$ has the significance already indicated, which is made to react with the primary or secondary amine with the formula (VII):

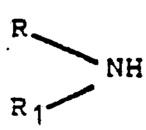

(VII)

in which R and $R_1$ have the significance already indicated, in order to obtain a product with the formula ($I_A$):

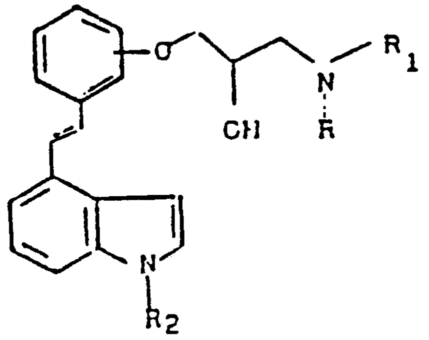

($I_A$)

in which R, $R_1$ and $R_2$ have the significance already indicated, which either is isolated and, if desired, salified, or is submitted to a hydrogenation in order to obtain a product with the formula ($I_B$):

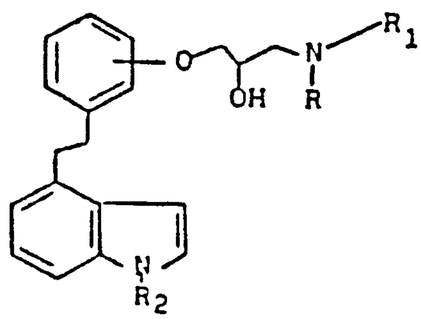

($I_B$)

in which R, $R_1$ and $R_2$ have the significance already indicated, which, if desired, is salified,
or the said product with the formula (IV) is made to react with the halogenide with the formula (V'):

$$Hal\text{-}(CH_2)_n\text{-}OR_3 \qquad (V')$$

in which
Hal and n have the significance already indicated and
$R_3$ represents a tosyl radical or a hydrogen atom, in order to obtain a product with the formula (VI'):

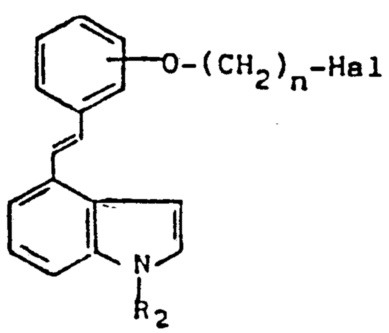

(VI')

in which n, Hal and $R_2$ have the significance already indicated, which is made to react with the amine with the formula (VII) defined above, in order to obtain a product with the formula ($I'_A$):

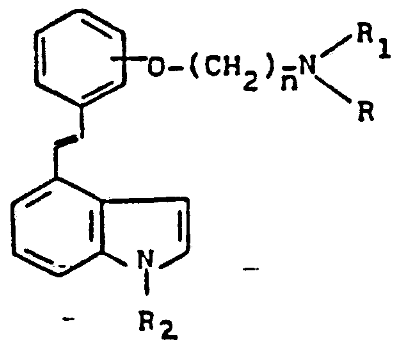

($I'_A$)

in which n, R, $R_1$ and $R_2$ have the significance already indicated, which either is isolated and, if desired, salified, or is submitted to a hydrogenation in order to obtain a product with the formula ($I'_B$):

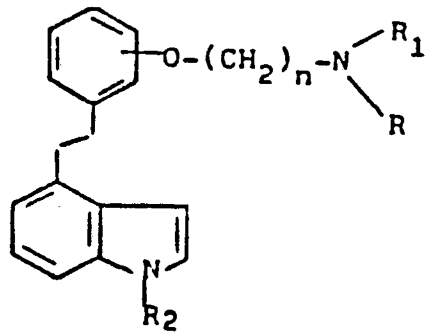

($I'_B$)

in which n, R, $R_1$ and $R_2$ have the significance already indicated, then the said products with the formulae ($I_A$), ($I_B$), ($I'_A$) and ($I'_B$) are submitted, if desired, to the action of a halogenation agent in order to obtain a product with the formula (VIII):

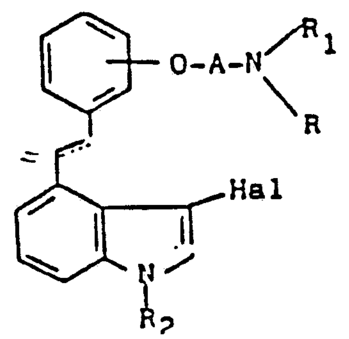

(VIII)

in which A, Hal, R, $R_1$, $R_2$ and the broken line have the significance already indicated, which is submitted to a hydrolysis in order to obtain a product with the formula ($I_C$):

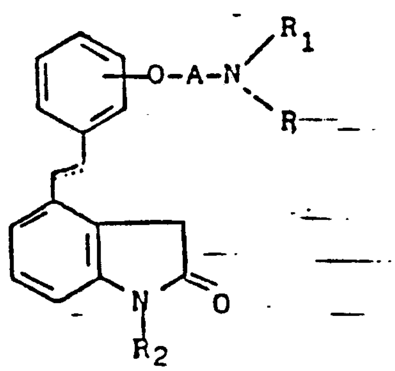

($I_C$)

in which A, R, $R_1$, $R_2$ and the broken line have the significance already indicated, which is isolated and, if desired, salified.

2. Process according to claim 1, characterized in that at the start a derivative with the formula (II) is used in which $R_2$ represents a hydrogen atom, a halogenide with the formula (V), then an amine with the formula (VII) in which R and $R_1$ represents a hydrogen atom or an alkyl radical, it being understood that R and $R_1$ cannot both at the same time represent a hydrogen atom.

3. Process according to claim 1, characterized in that at the start a derivative with the formula (II) is used in which $R_2$ represents a hydrogen atom, a halogenide with the formula (V'), then an amine with the formula (VII) in which R and $R_1$ represent a hydrogen atom or an alkyl radical.

4. Process according to any one of claims 1 to 3, characterized in that at the start a derivative with the formula (III) is used, in which the substituent OH is in the ortho position.

5. Process according to any one of claims 1 to 4, characterized in that derivatives with the formula (I) are prepared in which R represents a hydrogen atom and a and b together represent a carbon-carbon bond.

6. Process according to claim 1, characterized in that any one of the derivatives with the formula (I) are prepared, as defined in claim 1, of which the names follow:

- 1-[(1,1-dimethylethyl)amino]-3-[4-[2-(1H-indol-4-yl)ethenyl]-phenoxy]-2-propanol,
- 1-[(1,1-dimethylethyl)amino]-3-[2-[2-(1H-indol-4-yl)ethyl]-phenoxy]-2-propanol,
- 1,3-dihydro-4-[2-[2-[3-[(1,1-dimethylethyl)amino]-2-hydroxypropoxy]phenyl]ethenyl]-2H-indol-2-one,
- N-(1,1-dimethylethyl)-3-[2-[2-(1H-indol-4-yl)ethyl]-phenoxy]propanamine,

as well as their addition salts with mineral or organic acids.